(19) Europäisches Patentamt / European Patent Office / Office européen des brevets

(11) **EP 4 365 838 B1**

(12) **EUROPEAN PATENT SPECIFICATION**

(45) Date of publication and mention
of the grant of the patent:
**22.04.2026 Bulletin 2026/17**

(21) Application number: **22848626.2**

(22) Date of filing: **28.07.2022**

(51) International Patent Classification (IPC):
***G06T 7/33*** *(2017.01)*

(52) Cooperative Patent Classification (CPC):
**G06T 7/33; G06T 2207/10072; G06T 2207/10124;
G06T 2207/30012**

(86) International application number:
**PCT/CN2022/108552**

(87) International publication number:
**WO 2023/006021 (02.02.2023 Gazette 2023/05)**

(54) **REGISTRATION METHOD AND SYSTEM**

REGISTRIERUNGSVERFAHREN UND -SYSTEM

PROCÉDÉ ET SYSTÈME D'ENREGISTREMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.07.2021 CN 202110875674
17.02.2022 CN 202210147197**

(43) Date of publication of application:
**08.05.2024 Bulletin 2024/19**

(73) Proprietor: **Wuhan United Imaging Surgical Co.,
Ltd.**
**Wuhan, Hubei 430206 (CN)**

(72) Inventor: **FU, Chunmeng**
**Wuhan, Hubei 430073 (CN)**

(74) Representative: **Wang, Bo**
**Panovision IP**
**Ebersberger Straße 3**
**85570 Markt Schwaben (DE)**

(56) References cited:
CN-A- 109 325 971      CN-A- 111 429 491
CN-A- 112 233 155      CN-A- 112 308 765
CN-A- 113 570 648      CN-A- 114 529 594
US-A1- 2011 122 226    US-A1- 2014 334 709
US-A1- 2017 178 349

- KETCHA M D ET AL: "Multi-stage 3D-2D
  registration for correction of anatomical
  deformation in image-guided spine surgery",
  PHYSICS IN MEDICINE AND BIOLOGY,
  INSTITUTE OF PHYSICS PUBLISHING, BRISTOL
  GB, vol. 62, no. 11, 11 May 2017 (2017-05-11),
  pages 4604 - 4622, XP020317049, ISSN:
  0031-9155, [retrieved on 20170511], DOI: 10.1088/
  1361-6560/AA6B3E
- GAO CONG ET AL: "Fiducial-Free 2D/3D
  Registration for Robot-Assisted Femoroplasty",
  IEEE TRANSACTIONS ON MEDICAL ROBOTICS
  AND BIONICS, IEEE, vol. 2, no. 3, 27 July 2020
  (2020-07-27), pages 437 - 446, XP011806974, DOI:
  10.1109/TMRB.2020.3012460
- "Multiple View Geometry in Computer Vision -
  Second Edition", 1 January 2004, CAMBRIDGE
  UNIVERSITY PRESS, New York, ISBN:
  978-0-511-18618-9, article RICHARD HARTLEY
  ET AL: "Multiple View Geometry in Computer
  Vision", pages: i-xvi,1 - 655, XP055328738

## Description

### CROSS-REFERENCE TO RELATED APPLICATIONS

This application claims the priority to Chinese Patent Application No. 202210147197.6, titled "METHODS, SYSTEMS, COMPUTER DEVICES, AND STORAGE MEDIA FOR REGISTRATION," filed on February 17, 2022, and Chinese Patent Application No. 202110875674.6, titled "METHODS AND ELECTRONIC DEVICES FOR MULTI-SKELETON IMAGE REGISTRATION, AND MEDICAL NAVIGATION SYSTEMS," filed on July 30, 2021.

### TECHNICAL FIELD

The present disclosure relates to the field of computer-assisted surgery, and in particular, to methods and systems for performing registration between a three-dimensional (3D) image captured before a surgery and at least one two-dimensional (2D) image captured during the surgery.

### BACKGROUND

In a computer-assisted surgery, a doctor often needs to capture a 3D image (e.g., a computed tomography (CT) image or a magnetic resonance imaging (MRI) image) of a patient before a surgery, and determine a surgical plan based on the 3D image. Then, during a surgical process, the surgical plan determined based on the preoperative 3D image is mapped to a surgical space for guiding the intraoperative surgical process. In traditional techniques, a plurality of 2D images are captured during the surgery, a two dimensional-three dimensional (2D-3D) registration is performed between the preoperative 3D image and the plurality of 2D images, and an indirect mapping relationship between a 3D image space and the surgical space is established.

Existing 2D-3D registration techniques often have a large amount of complex human-computer interaction operations, including manual selection of a plurality of anatomical points in the plurality of 2D images and the 3D image, manual selection of a region to be registered, etc. The large amount of human-computer interaction operations and frequent manual operations affect the accuracy and efficiency of the registration. On the other hand, when the 2D-3D registration is performed on a joint part (e.g., a hip joint, an elbow joints, a knee joint, a spine, a finger joint, etc.) composed of a plurality of bones, a change of a joint posture of the patient causes relative motions of the bones in the joint part. Therefore, relative positions of the bones in the joint part when the preoperative 3D image is captured are inconsistent with relative positions of the bones in the joint part when the intraoperative 2D images are captured, which reduces the precision of the 2D-3D registration and the accuracy of surgical guidance. KETCHA M. D. et al. "Multi-stage 3D-2D registration for correction of anatomical deformation in image-guided spine surgery" (DOI: 10.1088/1361-6560/ AA6B3E), discloses a mapping of anatomical labels from a 3D preoperative image to a 2D intraoperative radiograph.

Therefore, it is desirable to provide a method and a system for performing registration between the 3D image captured before the surgery and at least one 2D image captured during the surgery.

### SUMMARY

One embodiment of the present disclosure provides a method for registration. The method may be implemented by at least one processor. The method may include obtaining a 3D image of a target object captured before a surgery and at least one 2D image captured during the surgery; obtaining a registered 3D image and a first transformation matrix between a 3D image coordinate system corresponding to the 3D image and a surgical space coordinate system corresponding to the surgery by performing posture transformation on the 3D image based on the at least one 2D image; determining a 2D target image corresponding to a target part of the target object in each of the at least one 2D image and determining a 3D target image corresponding to the target part in the registered 3D image; and obtaining a target transformation matrix by performing posture transformation on the registered 3D image based on the 3D target image and the 2D target image of each 2D image to optimize the first transformation matrix.

In some embodiments, a downsampled 3D image and at least one downsampled 2D image may be obtained by downsampling the 3D image and the at least one 2D image based on a preset multiplier. An adjusted downsampled 3D image may be obtained by adjusting a posture of the target object in the downsampled 3D image based on a preset step size. For each of the at least one 2D image, a first projection image corresponding to the 2D image may be obtained by projecting the adjusted downsampled 3D image based on a capturing posture of the 2D image. In response to determining that the at least one first projection image and the at least one downsampled 2D image satisfy a first preset condition, the registered 3D image may be obtained by upsampling the adjusted downsampled 3D image based on the preset multiplier. The first transformation matrix may be determined based on the posture transformation process from the 3D image to the registered 3D image.

In some embodiments, a first similarity degree may be determined based on the at least one first projection image and the at least one downsampled 2D image. In response to determining that the first similarity degree is larger than a similarity threshold, it may be determined that the at least one first projection image and the at least one downsampled 2D image satisfy the first preset condition.

In some embodiments, after for each of the at least one 2D image, obtaining the first projection image corresponding to the 2D image by projecting the adjusted downsampled 3D image based on the capturing posture of the 2D image, in response to determining that the at least one first projection image and the at least one downsampled 2D image do not satisfy the first preset condition, the posture of the target object in the adjusted downsampled 3D image may be adjusted based on the preset step size, and the operation of obtaining the first projection image corresponding to each of the at least one 2D image may be repeated until the at least one first projection image and the at least one downsampled 2D image satisfy the first preset condition.

In some embodiments, in response to determining that the at least one first projection image and the at least one downsampled 2D image satisfy the first preset condition and the preset multiplier does not satisfy a second preset condition, the 3D image may be updated by upsampling the adjusted downsampled 3D image based on the preset multiplier. The preset multiplier may be decreased, and the updated 3D image and the at least one 2D image may be downsampled based on the decreased preset multiplier to repeat the operation of obtaining the first projection image corresponding to each of the at least one 2D image until the at least one first projection image and the at least one downsampled 2D image satisfy the first preset condition and the decreased preset multiplier satisfies the second preset condition.

In some embodiments, in response to determining that the preset multiplier is equal to a threshold, it may be determined that the at least one first projection image and the at least one downsampled 2D image satisfy the second preset condition.

In some embodiments, a second transformation matrix between a 2D imaging device coordinate system and the surgical spatial coordinate system may be obtained. A third transformation matrix between the 3D image coordinate system and the 2D imaging device coordinate system may be obtained based on the posture transformation process from the 3D image to the registered 3D image. The first transformation matrix may be determined based on the second transformation matrix and the third transformation matrix.

In some embodiments, the 3D target image corresponding to the target part may be determined in the registered 3D image. For each of the at least one 2D image, a fourth transformation matrix between a 2D image coordinate system corresponding to the 2D image and the surgical spatial coordinate system may be obtained, and the 2D target image may be determined in the 2D image based on the 3D target image, the first transformation matrix, and the fourth transformation matrix.

In some embodiments, at least one 3D coordinate of at least one representative point of the target part in the 3D image coordinate system and a size parameter of the target part may be determined based on the 3D target image. At least one 2D coordinate of the at least one representative point in the 2D image coordinate system may be determined based on the at least one 3D coordinate, the first transformation matrix, and the fourth transformation matrix. The 2D target image may be determined in the 2D image based on the at least one 2D coordinate and the size parameter.

In some embodiments, a second transformation matrix between a 2D imaging device coordinate system and the surgical spatial coordinate system may be obtained. A fifth transformation matrix between the 2D imaging device coordinate system and the 2D image coordinate system may be obtained. The fourth transformation matrix may be determined based on the second transformation matrix and the fifth transformation matrix.

In some embodiments, for each of the at least one 2D image, a second projection image corresponding to the 2D image may be obtained by projecting the 3D target image based on a capturing posture of the 2D image. A second similarity degree may be determined based on the at least one second projection image and the at least one 2D target image. The target transformation matrix may be obtained by adjusting a posture of the target object in the registered 3D image based on the second similarity degree to optimize the first transformation matrix.

One embodiment of the present disclosure provides a system for registration. The system may include an image acquisition module, a posture transformation module, a target image determination module, and a target matrix determination module. The image acquisition module may be configured to obtain a 3D image of a target object captured before a surgery and at least one 2D image captured during the surgery. The posture transformation module may be configured to obtain a registered 3D image and a first transformation matrix between a 3D image coordinate system corresponding to the 3D image and a surgical space coordinate system corresponding to the surgery by performing posture transformation on the 3D image based on the at least one 2D image. The target image determination module may be configured to determine a 2D target image corresponding to a target part of the target object in each of the at least one 2D image and determine a 3D target image corresponding to the target part in the registered 3D image. The target matrix determination module may be configured to obtain a target transformation matrix by performing posture transformation on the registered 3D image based on the 3D target image and the 2D target image of each 2D image to optimize the first transformation matrix.

One embodiment of the present disclosure provides a non-transitory computer-readable storage medium storing one or more computer instructions. When a computer reads the one or more computer instructions from the storage medium, the computer may execute the method for registration.

According to some embodiments of the present disclosure, a rough registration between the 3D image and the at least one 2D image can be achieved by performing the posture transformation on the 3D image captured before the surgery based on the at least one 2D image captured during the surgery. A fine registration between the at least one 2D image and the 3D image can be achieved by further performing posture transformation on the roughly registered 3D image based on the 2D target image and the 3D target image corresponding to the target part. Through the two registration processes, no anatomical points need to be selected manually in the 3D image and the at least one 2D image, and no large amount of human-computer interaction operations need to be performed, which can significantly simplify the operational workflow and avoid errors caused by the large amount of human-computer interaction operations, thereby improving the accuracy of the registration between the 3D image and the at least one 2D image. Furthermore, the accuracy of the transformation matrix between the 3D image coordinate system and the surgical space coordinate system and the surgical guidance can be improved.

In addition, according to some embodiments of the present disclosure, the 2D target image corresponding to the target part can be determined from the at least one 2D image based on the 3D target image. Since the 2D image usually has a lower imaging definition than the 3D image and may include a surgical device, an optimal segmentation result can not be obtained by directly segmenting the target part from the 2D image. By determining the 2D target image based on the 3D target image, the accuracy of the target part segmentation can be improved.

## BRIEF DESCRIPTION OF THE DRAWINGS

The present disclosure will be further illustrated by way of exemplary embodiments, which will be described in detail through the accompanying drawings. These embodiments are not limiting, and in these embodiments the same numbering indicates the same structure, wherein:

FIG. 1 is a schematic diagram illustrating an application scenario of a registration system according to some embodiments of the present disclosure;

FIG. 2 is a block diagram illustrating an exemplary processing device according to some embodiments of the present disclosure;

FIG. 3 is a flowchart illustrating an exemplary registration process according to some embodiments of the present disclosure;

FIG. 4 is a flowchart illustrating an exemplary process of rough registration between a 3D image and at least one 2D image according to some embodiments of the present disclosure;

FIG. 5 is a flowchart illustrating an exemplary process for generating a 3D target image and at least one 2D target image according to some embodiments of the present disclosure;

FIG. 6 is a flowchart illustrating an exemplary process for obtaining a target transformation matrix by optimizing a first transformation matrix according to some embodiments of the present disclosure;

FIGs. 7A and 7B are schematic diagrams illustrating 2D images corresponding to a first capturing posture according to some embodiments of the present disclosure;

FIGs. 8A and 8B are schematic diagrams illustrating 2D images corresponding to a second capturing posture according to some embodiments of the present disclosure;

FIG. 9 is a schematic diagram illustrating a registration process according to some embodiments of the present disclosure;

FIG. 10 is a schematic diagram illustrating a registration process according to some embodiments of the present disclosure; and

FIG. 11 is a schematic diagram illustrating a structure of a registration system according to some embodiments of the present disclosure.

## DETAILED DESCRIPTION

In order to more clearly illustrate the technical solutions of the embodiments of the present disclosure, the accompanying drawings to be used in the description of the embodiments will be briefly described below. Obviously, the accompanying drawings in the following description are only some examples or embodiments of the present disclosure, and that the present disclosure may be applied to other similar scenarios in accordance with these drawings without creative labor for those of ordinary skill in the art. Unless obviously obtained from the context or the context illustrates otherwise, the same numeral in the drawings refers to the same structure or operation.

It should be understood that "system," "device," "unit," and/or "module" as used herein is a way to distinguish between different components, elements, parts, sections, or assemblies at different levels. However, these words may be replaced by other expressions if other words accomplish the same purpose.

As indicated in the present disclosure and in the claims, unless the context clearly suggests an exception, the words "one," "a," "a kind of," and/or "the" do not refer specifically to the singular but may also include the plural. In general, the terms "including" and "comprising" suggest only the inclusion of clearly identified steps and elements, which do not constitute an exclusive list, and the method or device may also include other steps or elements.

The present disclosure uses flowcharts to illustrate the operations performed by the system according to some embodiments of the present disclosure. It should be understood that the operations described herein are not necessarily executed in a specific order. Instead, they may be executed in reverse order or simultaneously. Additionally, other operations may be added to these processes or certain steps may be removed.

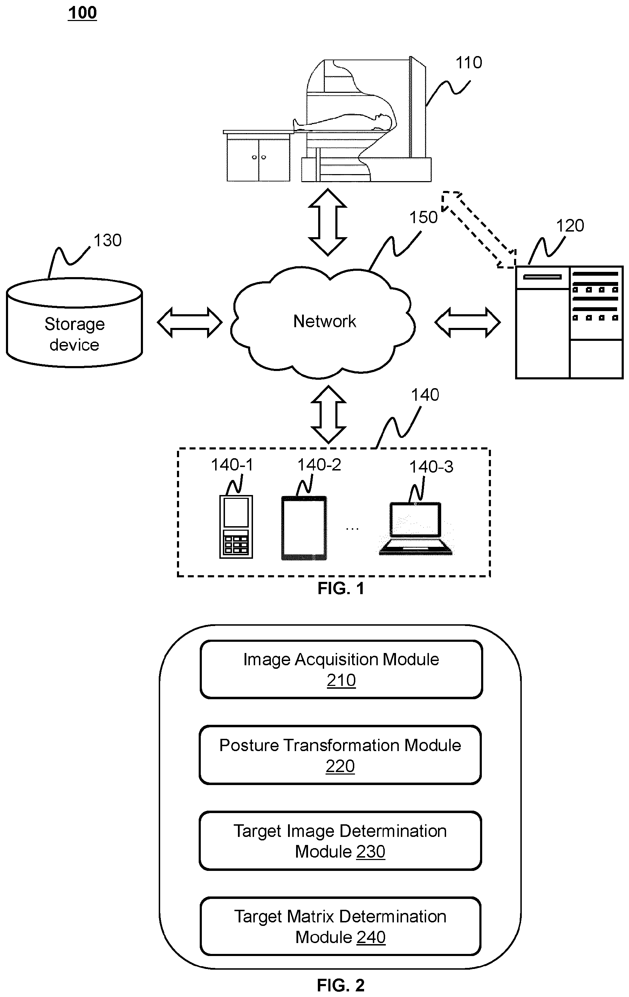

FIG. 1 is a schematic diagram illustrating an application scenario of an exemplary registration system according to some embodiments of the present disclosure.

As shown in FIG. 1, in some embodiments, a registration system 100 may include a medical imaging device 110, a processing device 120, a storage device 130, at least one terminal 140, and a network 150. The registration system 100 may implement the method(s) and/or process(es) disclosed in the present disclosure through the processing device 120 to perform a 2D-3D registration on images obtained by the medical imaging device 110. In the present disclosure, the 2D-3D registration refers to a registration between a 3D image of a target object captured before a surgery and at least one 2D image of the target object captured during the surgery. During the registration process, a posture corresponding to the 3D image may be adjusted (e.g., through translation and/or rotation) so that a posture of the target object in an adjusted 3D image (i.e., a registered 3D image) is the same as a posture of the target object when the at least one 2D image is captured. Through the 2D-3D registration, a mapping relationship may be established between a 3D image space (also referred to as a 3D image coordinate system) and a surgical space (also referred to as a surgical space coordinate system). Therefore, a surgical plan determined based on the 3D image can be accurately converted into the surgical space, thereby ensuring the smooth surgery and improving the surgical effect.

The medical imaging device 110 refers to a device in medicine that use different techniques to reproduce an internal structure of the target object (e.g., a human body, an animal, etc.) as an image. In some embodiments, the medical imaging device 110 may be any medical devices capable of imaging or treating a specified body part of a patient, such as a computed tomography (CT) device, a magnetic resonance imaging (MRI) device, a positron emission computed tomography (PET) device, a direct digit radiography (DDR) device, an X-ray imaging device, etc. The above examples of the medical imaging device 110 are merely provided for purposes of illustration, and do not limit the scope of the medical imaging device 110.

In some embodiments, the medical imaging device 110 may be configured to capture 2D and/or 3D image(s) of the target object. In some embodiments, the medical imaging device 110 may include a first medical imaging device and a second medical imaging device. The first medical imaging device may be configured to capture the 3D image(s), and the second medical imaging device may be configured to capture the 2D image(s). For example, the first imaging device may be a CT device or an MRI device configured to capture a 3D image of the target object before a surgery, and the second imaging device may be an X-ray imaging device configured to capture 2D images of the target object during the surgery. The 3D image may be used to determine a surgical plan (e.g., a surgical path), the 2D images may be used to register with the 3D image to obtain a transformation matrix, and the transformation matrix may be used to map the surgical plan into the surgical space. In some embodiments, the medical imaging device 110 may include a single device capable of capturing both the 3D image and the 2D images.

In some embodiments, data and/or information (e.g., the image(s) captured by the medical imaging device 110) may be stored in the storage device 130. The medical imaging device 110 may receive instruction(s) issued by a doctor via the at least one terminal 140, and perform relevant operation(s) based on the instruction(s). For example, the relevant operation(s) may include irradiation imaging, etc. In some embodiments, the medical imaging device 110 may exchange the data and/or information with other components (e.g., the processing device 120, the storage device 130, the at least one terminal 140) of the registration system 100 via the network 150. In some embodiments, the medical imaging device 110 may be directly connected to the other components of the registration system 100. In some embodiments, one or more components (e.g., the processing device 120, the storage device 130) of the registration system 100 may be included within the medical imaging device 110.

The processing device 120 may process data and/or information obtained from other devices or components of the registration system 100, and execute the registration method disclosed in some embodiments of the present disclosure based on the data, information, and/or a processing result, so as to achieve one or more functions described in some embodiments of the present disclosure. For example, the processing device 120 may perform the 2D-3D registration based on the 2D images and the 3D image captured by the medical imaging device 110, thereby determining a transformation relationship between the 3D image space and the surgical space. In some embodiments, the processing device 120 may send processed data (e.g., a transformation matrix between coordinate systems, a projection image, etc.) to the storage device 130 for storage. In some embodiments, the processing device 120 may retrieve pre-stored data and/or information (e.g., the 2D images, the 3D image, etc.) from the storage device 130 for executing the registration method (e.g., the 2D-3D registration, etc.) disclosed in some embodiments of the present disclosure.

In some embodiments, the processing device 120 may include one or more sub-processing devices (e.g., a single-core processing device or a multi-core/multi-threaded processing device). Merely by way of example, the processing device 120 may include a central processing unit (CPU), an application-specific integrated circuit (ASIC), an application-specific instruction processor (ASIP), a graphics processing unit (GPU), a physics processing unit (PPU), a digital signal processor (DSP), a field-programmable gate array (FPGA), a programmable logic device (PLD), a controller, a microcontroller unit, a reduced instruction set computer (RISC), a microprocessor, or the like, or any combination thereof.

The storage device 130 may store data or information generated by other devices. In some embodiments, the storage device 130 may store data and/or information captured by the medical imaging device 110, such as the 2D images, the 3D image, etc. In some embodiments, the storage device 130 may store data and/or information processed by the processing device 120, such as the transformation matrix between the coordinate systems, the projection image, etc. The storage device 130 may include one or more storage components, and each storage component may be an independent device or a portion of other device. The storage device may be local or implemented via the cloud.

The at least one terminal 140 may control operation(s) of the medical imaging device 110. For example, the doctor may issue an operational instruction to the medical imaging device 110 via the at least one terminal 140 to cause the medical imaging device 110 to perform a specified operation. For example, the specified operation may include imaging the specified body part of the patient. In some embodiments, the at least one terminal 140 may direct the processing device 120 through instruction(s) to execute the registration method disclosed in some embodiments of the present disclosure. In some embodiments, the at least one terminal 140 may receive the registered 3D image from the processing device 120, so that a user can perform an effective and targeted detection and/or treatment on the patient. In some embodiments, the at least one terminal 140 may be any device with an input and/or output function, such as a mobile device 140-1, a tablet computer 140-2, a laptop computer 140-3, a desktop computer, or the like, or any combination thereof.

The network 150 may connect various components of the registration system 100 and/or connect the registration system 100 to an external resource part. The network 150 may enable communications between the various components of the registration system 100 and communications between the various components and other components outside the registration system 100 to facilitate data and/or information exchange. In some embodiments, one or more components (e.g., the medical imaging device 110, the processing device 120, the storage device 130, the at least one terminal 140) of the registration system 100 may send data and/or information to other components via the network 150. In some embodiments, the network 150 may be any one or more of a wired network or a wireless network.

It should be noted that the above description is merely provided for purposes of illustration, and is not intended to limit the scope of the present disclosure. For persons having ordinary skills in the art, various variations and modifications may be conducted under the teaching of the present disclosure. The features, structures, methods, and other features of the exemplary embodiments described in the present disclosure can be combined in various manners to obtain additional and/or alternative exemplary embodiments. For example, the processing device 120 may be run based on a cloud computing platform, such as a public cloud, a private cloud, a community cloud, a hybrid cloud, etc. However, such variations and modifications do not depart from the scope of the present disclosure.

FIG. 2 is a block diagram illustrating an exemplary processing device according to some embodiments of the present disclosure.

As shown in FIG. 2, in some embodiments, the processing device 120 may include an image acquisition module 210, a posture transformation module 220, a target image determination module 230, and a target matrix determination module 240.

The image acquisition module 210 may be configured to obtain a 3D image of a target object (e.g., a human body, an animal, etc.) captured before a surgery and at least one 2D image captured during the surgery.

The posture transformation module 220 may be configured to obtain a registered 3D image and a first transformation matrix between a 3D image coordinate system corresponding to the 3D image and a surgical space coordinate system corresponding to the surgery by performing posture transformation on the 3D image based on the at least one 2D image.

The target image determination module 230 may be configured to determine a 2D target image corresponding to a target part of the target object in each of the at least one 2D image and determine a 3D target image corresponding to the target part in the registered 3D image. In some embodiments, the target part may include at least one of a target spine, a target hip joint, a target elbow joint, a target knee joint, a target finger joint, etc.

The target matrix determination module 240 may be configured to obtain a target transformation matrix by performing posture transformation on the registered 3D image based on the 3D target image and the 2D target image of each 2D image to optimize the first transformation matrix.

In some embodiments, the processing device 120 may further include a path information acquisition module and a guidance information generation module (not shown in FIG. 2). The path information acquisition module may be configured to obtain surgical path information corresponding to the 3D image. The guidance information generation module may be configured to generate surgical guidance information by converting the surgical path information into the surgical space coordinate system based on the target transformation matrix.

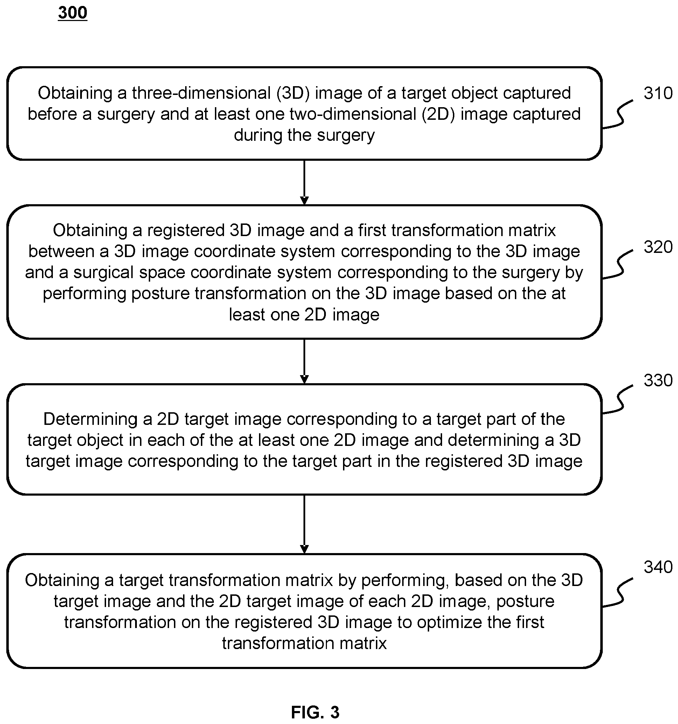

More descriptions regarding the above modules may be found in FIGs. 3-5, and relevant descriptions thereof.

FIG. 3 is a flowchart illustrating an exemplary registration process according to some embodiments of the present disclosure.

In some embodiments, a process 300 may be executed by the registration system 100. For example, the process 300 may be stored in the form of one or more instruction sets (e.g., an application) in a storage device (e.g., the storage device 130). In some embodiments, the processing device 120 (e.g., one or more of the modules shown in FIG. 2) may execute the one or more instruction sets and direct one or more components of the registration system 100 to perform the process 300. As shown in FIG. 3, the process 300 may include the following operations.

In 310, a 3D image of a target object captured before a surgery and at least one 2D image of the target object captured during the surgery may be obtained. In some embodiments, operation 310 may be performed by the image acquisition module 210.

The target object refers to an object that needs a surgery or a detection, such as an organism, a phantom, etc. In some embodiments, the target object may include a human body or a portion of the human body. For example, the target object may include a patient or a portion (e.g., a spine, a hip joint, an elbow joint, a knee joint, a finger, etc.) of the patient who needs the surgery. In some embodiments, the target object may include a target part. The target part refers to a body region (e.g., an organ, a tissue, etc.) in the target object that needs the surgery and/or the detection. For example, the target part may include a target spine, a target hip joint, a target elbow joint, a target knee joint, a target finger joint, or the like.

The 3D image refers to an image obtained by capturing the target object using a 3D medical imaging device, such as a CT image, an MRI image, a PET image, etc. In some embodiments, the image acquisition module 210 may obtain the 3D image by directing the 3D medical imaging device to scan the target object before the surgery. The 3D image may be used for detection, diagnosis, preoperative preparation, intraoperative path localization, etc.

The 2D image refers to an image obtained by capturing the target object using a 2D medical imaging device, such as a DDR image, an X-ray image, etc. In some embodiments, the image acquisition module 210 may obtain the 2D image by directing the 2D medical imaging device to scan the target object during the surgery. The 2D image may be used for performing registration with the 3D image, such that a posture of the target object in the 3D image is the same as a posture of the target object during the surgery. In addition, a transformation matrix between a 3D image space and a surgical space may be obtained, and the transformation matrix may be used to convert a surgical plan into the surgical space. In some embodiments, the 2D image may include at least two 2D images. Capturing angles of the at least two 2D images may be different. For example, the at least two 2D images may include an anteroposterior 2D image and a lateral 2D image. When the anteroposterior 2D image is captured, a radiation source is positioned directly in front of the target object for irradiation. When the lateral 2D image is captured, the radiation source is positioned at a side of the target object for irradiation. In some embodiments, the at least one 2D image may include at least two 2D images, and an angle between any two of the at least two 2D images may be within a preset angle range. The angle between two 2D images refers to an angle between two sets of rays emitted by the 2D medical imaging device when capturing the two 2D images, i.e., an angle between imaging planes of the two 2D images. In some embodiments, the preset angle range may be from 50 degrees to 130 degrees.

In some embodiments, the image acquisition module 210 may obtain the 3D image of the target object captured before the surgery and the at least one 2D image captured during the surgery in other manners. For example, the image acquisition module 210 may obtain the 3D image of the target object captured before the surgery and the at least one 2D image captured during the surgery from various data sources (e.g., a storage device, etc.).

In 320, a registered 3D image and a first transformation matrix between a 3D image coordinate system and a surgical space coordinate system may be obtained by performing posture transformation on the 3D image based on the at least one 2D image. In some embodiments, operation 320 may be performed by the posture transformation module 220.

The posture transformation on the 3D image in operation 320 may be performed based on an overall analysis of the 3D image, which may be considered as a process of a rough registration on the 3D image. By performing the rough registration, a posture (i.e., a position and a pose) of the target object in the 3D image may be adjusted to be approximately the same as a posture (i.e., a current posture during the surgery) of the target object when capturing the at least one 2D image. The registered 3D image refers to an image obtained by performing the rough registration on the 3D image. The first transformation matrix refers to a transformation matrix between the 3D image coordinate system corresponding to the 3D image and the surgical space coordinate system corresponding to the surgery, which is determined through the rough registration.

The surgical space coordinate system (also referred to as the surgical space or a surgical coordinate system) refers to a coordinate system generated with a preset position as a reference point for guiding the surgery. In some embodiments, the surgical space coordinate system may include a coordinate system established based on an optical tracking system (OTS). The OTS may capture an entire surgical scene using an optical imaging technique. For example, the OTS may include a binocular camera. The surgical space coordinate system may be established with a point on the binocular camera as a coordinate origin, a horizontal direction as an x-axis, a vertical direction as a z-axis, and a front-rear direction as a y-axis.

The 3D image coordinate system (also referred to as the 3D image space) refers to a 3D coordinate system established based on the 3D image. For example, if the target object in the 3D image is a patient, the 3D image coordinate system may be established with a center point of the patient as a coordinate origin, a left-right direction of the patient as an x-axis, a front-rear direction as a y-axis, and a head-feet direction as a z-axis.

In some embodiments, the processing device 120 may obtain an adjusted 3D image by adjusting the posture of the target object in the 3D image (also referred to as the posture corresponding to the 3D image). Furthermore, the processing device 120 may obtain a first projection image corresponding to each of the at least one 2D image by projecting the adjusted 3D image at different projection postures. For each of the at least one 2D image and the first projection image corresponding to the 2D image, a capturing posture of the 2D image is the same as a projection posture of the first projection image. The capturing posture refers to a posture of the 2D medical imaging device when the 2D medical imaging device captures the 2D image, and the projection posture refers to a posture of a virtual imaging device when the projection image is obtained through projection. For example, when capturing a 2D image, a C-arm posture (i.e., position coordinates of a radiation source and a center of an imaging plane of the C-arm) in the surgical coordinate system may be obtained through an OTS device. In the 3D image coordinate system, position coordinates of a radiation source and a center of an imaging plane of a virtual C-arm may be set to be equal to the position coordinates of the radiation source and the center of the imaging plane of the C-arm in the surgical coordinate system, such that the projection posture is the same as the capturing posture. After the first projection image is obtained, the processing device 120 may determine a first similarity degree based on the at least one 2D image and the at least one first projection image corresponding to the at least one 2D image, and determine whether to continue adjusting a posture corresponding to the adjusted 3D image based on the first similarity degree. In some embodiments, a similarity degree between each 2D image and the corresponding first projection image may be determined, and the first similarity degree may be obtained based on all determined similarity degrees. For example, the first similarity degree may be one of a sum, an average, a maximum value, etc., of all the determined similarity degrees. If the first similarity degree is relatively small, the posture corresponding to the adjusted 3D image may be further adjusted to update the adjusted 3D image. The process of determining the similarity degrees may be repeated until it is determined that no further adjustments of the posture corresponding to the adjusted 3D image need based on the similarity degrees, and then the registered 3D image may be obtained. More descriptions regarding the obtaining the registered 3D image by performing the posture transformation on the 3D image may be found in operations 410-460 and relevant descriptions thereof.

In some embodiments, the processing device 120 may determine the first transformation matrix based on the registered 3D image. For instance, during the rough registration process, the posture corresponding to the 3D image may be adjusted based on a rotation step size and a translation step size, and the first transformation matrix may be determined based on a total rotation step size and a total translation step size during the entire adjustment process. More descriptions regarding the determination of the first transformation matrix based on the registered 3D image may be found in operation 470 and relevant descriptions thereof.

In the rough registration, the projection images of the 3D image at different projection postures may not completely align with the corresponding 2D images. Taking the spine as an example, the spine as a whole is not rigid, but each vertebra in the spine is rigid. Since a relative movement of the spine usually occurs before and during the surgery, even though the spine as a whole in the registered 3D image after the rough registration is aligned with the spine in the surgical space coordinate system, one or more vertebrae may still not be fully aligned. If surgical guidance is performed based on the registered 3D image, it may affect the surgical effect or even lead to a surgical failure.

In some embodiments, after the rough registration, a fine registration between the registered 3D image and the at least one 2D image may be performed through subsequent operations 330 and 340. Different from the rough registration, the posture corresponding to the registered 3D image in the fine registration may be adjusted based on a target part.

In 330, a 2D target image corresponding to the target part of the target object may be determined in each of the at least one 2D image and a 3D target image corresponding to the target part of the target object may be determined in the registered 3D image. In some embodiments, operation 330 may be performed by the target image determination module 230.

A 2D target image refers to a 2D image that includes the target part, such as a 2D image of the target spine, etc. The 3D target image refers to a 3D image that includes the target part, such as a 3D image of the target spine. In some embodiments, the 2D target image(s) and/or the 3D target image may only include an image of the target part (or a small amount of a surrounding region of the target part), and the original 2D image(s) and the original 3D image (e.g., the at least one 2D image captured during the surgery and the 3D image captured before the surgery) may include other body part(s) besides the target part. For example, if the target part is a L3 vertebra, the 2D target image(s) and 3D target image may only include the L3 vertebra, and the original 2D image(s) and the original 3D image may include L1, L2, L3, L4, and L5 vertebrae.

In some embodiments, the processing device 120 may determine the 2D target image(s) and the 3D target image corresponding to the target part of the target object in the at least one 2D image and the registered 3D image, respectively.

For example, a first image segmentation algorithm (e.g., a 2D image segmentation model) may be used to segment a portion corresponding to the target part from the at least one 2D image, thereby generating the 2D target image(s). A second image segmentation algorithm (e.g., a 3D image segmentation model) may be used to segment a portion corresponding to the target part from the registered 3D image, thereby generating the 3D target image.

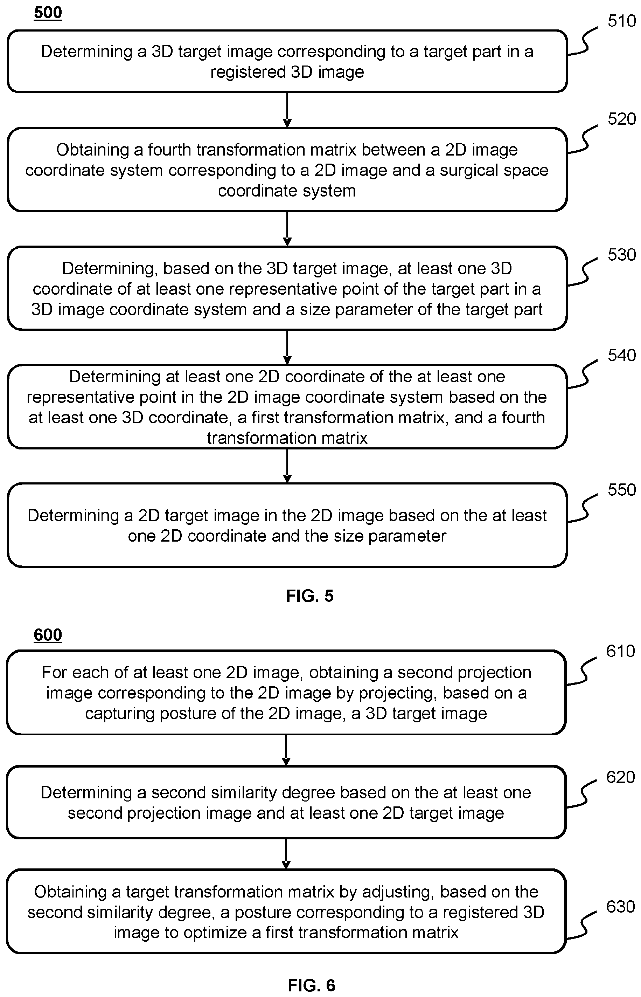

In some embodiments, the processing device 120 may determine the 3D target image including the target part in the registered 3D image. For each of the at least one 2D image, a fourth transformation matrix between a 2D image coordinate system corresponding to the 2D image and the surgical space coordinate system may be obtained, and the 2D target image(s) may be determined based on the 3D target image, the first transformation matrix, and the fourth transformation matrix. That is, the 3D target image may be determined, and then the 2D target image(s) may be determined based on the 3D target image. The 2D image coordinate system (also referred to as a 2D image space) refers to a 2D coordinate system established based on the 2D image. For example, the 2D image coordinate system may be established with an upper-left corner of the 2D image as a coordinate origin, a left-to-right direction of the 2D image as a positive x-axis, and a top-to-bottom direction as a positive y-axis. More descriptions regarding the determination of the 2D target image(s) and the 3D target image may be found in FIG. 5 and relevant descriptions thereof.

In 340, a target transformation matrix may be obtained by performing posture transformation on the registered 3D image based on the 3D target image and the 2D target image of each of the at least one 2D image to optimize the first transformation matrix. In some embodiments, operation 340 may be performed by the target matrix determination module 240.

The target transformation matrix refers to a transformation matrix between the 3D image coordinate system corresponding to the 3D target image and the surgical space coordinate system corresponding to the surgery, which is obtained based on a fine registration result.

In some embodiments, for each of the at least one 2D image, the processing device 120 may obtain a second projection image corresponding to the 2D image by project the 3D target image based on the capturing posture of the 2D image. Furthermore, the processing device 120 may determine a second similarity degree based on the at least one second projection image and the at least one 2D target image. Then, the processing device 120 may obtain the target transformation matrix by adjusting the posture corresponding to the registered 3D image based on the second similarity degree to optimize the first transformation matrix. More descriptions regarding the obtaining the target transformation matrix may be found in FIG. 6 and relevant descriptions thereof.

In some embodiments, the processing device 120 may further obtain surgical path information corresponding to the 3D image. For example, the surgical path information may include coordinate(s) of target point(s) of the target object in the 3D image coordinate system that a surgical instrument passes through during the surgery. The processing device 120 may transform the surgical path information into the surgical space coordinate system based on the target transformation matrix to generate surgical guidance information. For example, the processing device 120 may convert the coordinate(s) of the target point(s) in the 3D image coordinate system into coordinate(s) in the surgical space coordinate system based on the target transformation matrix, and designate the coordinate(s) in the surgical space coordinate system as the surgical guidance information. As another example, the surgical guidance information may include a determination result of whether position(s) of the surgical instrument are correct and/or prompt information for guiding movement(s) of the surgical instrument.

According to some embodiments of the present disclosure, the registered 3D image and the transformation matrix between the 3D image coordinate system and the surgical space coordinate system can be obtained by performing the posture transformation on the 3D image based on the at least one 2D image obtained during the surgery, such that the rough registration between the 3D image and the at least one 2D image can be achieved. Furthermore, the fine registration between the 3D image and the at least one 2D image can be achieved by further performing posture transformation on the roughly registered 3D image to optimize the first transformation matrix. Through the two registrations, the accuracy of the registration can be improved, thereby improving the accuracy of the target transformation matrix between the determined 3D image space and the surgical space and the surgical guidance. In addition, the 2D-3D registration method described in the present disclosure can be implemented with minimal or no human intervention, which needs no human-computer interaction (e.g., manually selecting anatomical points in the 3D image and the 2D image), thereby significantly simplifying the operational workflow and avoiding errors caused by the large amount of human-computer interaction. Therefore, the accuracy of the registration between the 3D image and the 2D images can be improved.

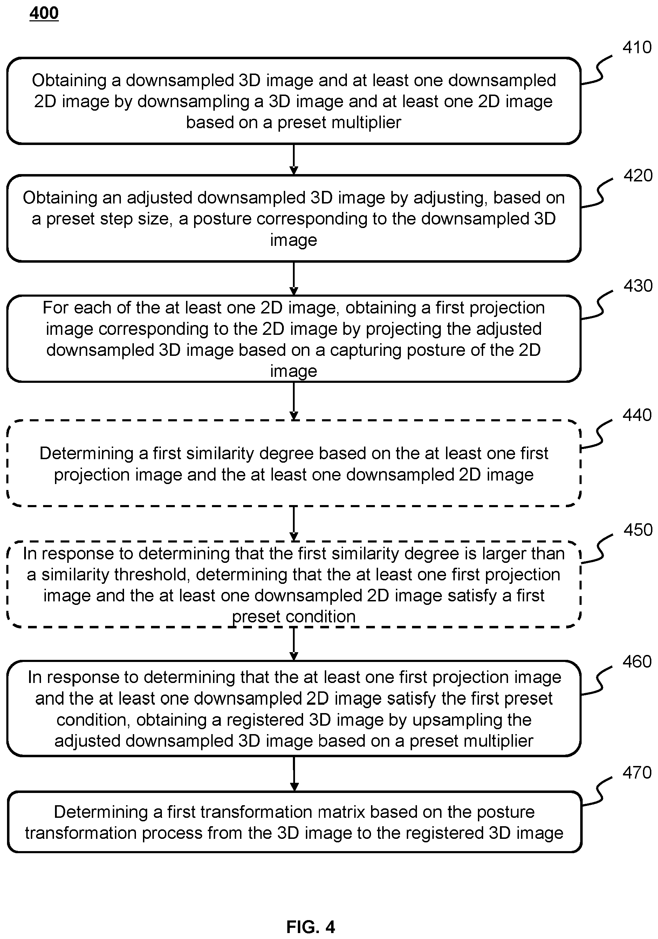

FIG. 4 is a flowchart illustrating an exemplary process of rough registration between a 3D image and at least one 2D image according to some embodiments of the present disclosure.

In some embodiments, the posture transformation module 220 may obtain a registered 3D image and determine a first transformation matrix between a 3D image coordinate system and a surgical space coordinate system by performing posture transformation on a 3D image based on at least one 2D image through the process 400. The process 400 may be performed to achieve at least part of operation 320 as described in connection with FIG. 3. As shown in FIG.4, the process 400 may include the following operations.

In 410, a downsampled 3D image and at least one downsampled 2D image may be obtained by downsampling the 3D image and the at least one 2D image based on a preset multiplier.

In some embodiments, the posture transformation module 220 may downsample the 3D image and the at least one 2D image based on the preset multiplier. That is, sizes of the 3D image and the at least one 2D image may be reduced by the preset multiplier. The preset multiplier may be any arbitrary value, such as 8, 4, 2, etc.

In 420, an adjusted downsampled 3D image may be obtained by adjusting a posture corresponding to the downsampled 3D image based on a preset step size.

In some embodiments, the preset step size may include a preset rotation step size and/or a preset translation step size. The posture transformation module 220 may rotate the downsampled 3D image based on the preset rotation step size and/or translate the downsampled 3D image based on the preset translation step size. The preset rotation step size may be any arbitrary angle, such as 10 degrees, 5 degrees, 2 degrees, etc. The preset translation step size may be any arbitrary length, such as 20 millimeters, 10 millimeters, 5 millimeters.

In some embodiments, the preset multiplier and preset step size may be default settings of the registration system 100, set by a user, or determined by the posture transformation module 220 based on actual requirement(s). In some embodiments, the preset multiplier and the preset step size may have a specific corresponding relationship. For example, a preset multiplier of 8 may correspond to a preset rotation step size of 10 degrees and a preset translation step size of 20 millimeters. A preset multiplier of 4 may correspond to a preset rotation step size of 5 degrees and a preset translation step size of 10 millimeters. A preset multiplier of 2 may correspond to a preset rotation step size of 5 degrees and a preset translation step size of 5 millimeters.

In 430, for each of the at least one 2D image, a first projection image corresponding to the 2D image may be obtained by projecting the adjusted downsampled 3D image based on a capturing posture of the 2D image.

In some embodiments, for each of the at least one 2D image, the posture transformation module 220 may project the adjusted downsampled 3D image at a projection posture to obtain the first projection image corresponding to the projection posture. The projection posture of the first projection image is the same as the capturing posture of the 2D image. Since downsampling does not change the capturing posture of the 2D image, the capturing posture of the 2D image is the same as the capturing posture of the downsampled 2D image corresponding to the 2D image, and then the projection posture of the first projection image corresponding to the 2D image is also the same as the capturing posture of the downsampled 2D image corresponding to the 2D image. Merely by way of example, if the 2D image includes an anteroposterior 2D image and a lateral 2D image, the posture transformation module 220 may obtain an anteroposterior first projection image and a lateral first projection image by projecting the adjusted downsampled 3D image from an anteroposterior position and a lateral position, respectively.

In some embodiments, the posture transformation module 220 may project the adjusted downsampled 3D image using technique(s) (e.g., digital reconstruction radiograph (DRR) projection) to obtain the first projection image.

In some embodiments, after the at least one first projection image corresponding to the at least one 2D image is obtained, whether the at least one first projection image and the at the least one downsampled 2D image satisfy a preset condition may be determined. In some embodiments, whether the at least one first projection image and the at least one downsampled 2D image satisfy the preset condition may be determined through operations 440 and 450.

In 440, a first similarity degree may be determined based on the at least one first projection image and the at least one downsampled 2D image.

The first similarity degree is used to measure a similarity degree between the at least one first projection image and their corresponding downsampled 2D image. In some embodiments, the posture transformation module 220 may determine a similarity degree between each of the at least one first projection image and the downsampled 2D image corresponding to the first projection image in various manners, and obtain the first similarity degree based on all determined similarity degrees. For example, the first similarity degree may be one of a sum, an average, a maximum value, etc., of all the determined similarity degrees.

In some embodiments, the similarity degree between a first projection image and the downsampled 2D image corresponding to the first projection image may be determined through various similarity algorithms. Exemplary similarity algorithms may include mutual information (MI) algorithm, pattern intensity (PI) algorithm, gradient difference (GD) algorithm, etc.

MI is used to represent a statistical correlation between two systems or an amount of information contained in one system about another system. In some embodiments, MI between two images may be represented by the following equation:

$$S = \sum_{x,y} p(x,y) \log \frac{p(x,y)}{p(x)p(y)}, \qquad (1)$$

where $p(x)$ and $p(y)$ represent marginal probability distributions of the two images to be registered, respectively; $p(x, y)$ represents a joint probability distribution of the two images to be registered; and S represents a mutual information value between the two images to be registered.

PI is determined based on a difference image between two images to be registered. A target to be registered is referred to as a "pattern." For instance, $I_{dif}$ may be determined by subtracting one image from another image. When a registration status is achieved, a pattern to be registered in $I_{dif}$ may disappear, and an intensity of the pattern decreases to a minimum. In some embodiments, the PI between the two images may be represented by the following equation:

$$\mathrm{P}_{r,\sigma} = \sum_{i,j} \sum_{d^2 \leq r^2} \frac{\sigma^2}{\sigma^2 + \left(I_{dif}(i,j) - I_{dif}(v,w)\right)^2}, \qquad (2)$$

where $I_{dif}$ represents a subtracted image between the two images to be registered; $r$ represents a radius of an effective calculation region of a pattern intensity for each pixel; $i$, j, $v$, and $w$ represent pixel coordinates in the subtracted image; $\mathrm{P}_{r,\sigma}$ represents a final pattern intensity value; $I_{dif}(i, j)$ represents a pixel value of a specific coordinate in the subtracted image of the two images to be registered; $I_{dif}(v, w)$ represents a pixel value of a coordinate within a neighborhood of the specific coordinate in the subtracted image of the two images to be registered; $d$ represents a radius; $d^2$ represents an effective region of a circle with $d$ as the radius, wherein $d^2 = (i - v)^2 + (j - w)^2$; and $\sigma$ refers to a constant representing a weight, wherein $\sigma$ is used to eliminate noise interference.

GD is also implemented based on a difference image, and the difference image is obtained from a gradient image. For instance, four gradient images may be generated by processing the two images using horizontal and vertical Sobel operators. The four gradient images may represent change rates of the two images to be registered in directions of two orthogonal coordinate axes, respectively. In some embodiments, a gradient difference measurement between the two images may be represented by the following equation:

$$G(s) = \sum_{i,j} \frac{A_v}{A_v + \left(I_{diffV}(i,j)\right)^2} + \sum_{i,j} \frac{A_h}{A_h + \left(I_{diffH}(i,j)\right)^2}, \qquad (3)$$

$$I_{diffV}(i,j) = \frac{dI_{fl}}{di} - s\frac{dI_{DRR}}{di}, \qquad (4)$$

where $\frac{dI_{fl}}{di}$ represents a gradient image of a 2D image to be registered in a horizontal direction; $\frac{dI_{DRR}}{di}$ represents a gradient image of a DRR image in the horizontal direction; $I_{diffV}$ represents a subtracted image between the gradient image of the 2D image to be registered and the gradient image of the DRR image; $s$, $A_v$, and $A_h$ represent function weights, G($s$) represents a final gradient difference, $I_{diffV}(i, j)$ represents a pixel value at a coordinate $(i, j)$ of the subtracted image between the gradient image of the 2D image to be registered and the DRR image in the horizontal direction; and $I_{diffH}$ represents a pixel value at the coordinate $(i, j)$ of the subtracted image between gradient image of the 2D image to be registered and the DRR image in a vertical direction.

According to some embodiments of the present disclosure, by using the DRR projection, the 3D image can be projected to 2D images with the same count as the 2D images to be registered, and the similarity degree may be determined, which can improve the acquisition of the similarity degree, thereby improving the accuracy of the registration.

In 450, in response to determining that the first similarity degree is larger than a similarity threshold, it may be determined that the at least one first projection image and the at least one downsampled 2D image satisfy the first preset condition.

The similarity threshold may be any numerical value. In some embodiments, the similarity threshold may be default settings of the registration system 100, set by the user, or determined by the posture transformation module 220 according to actual requirement(s). In some embodiments, the similarity threshold and the preset multiplier have a corresponding relationship. For example, when the at least one 2D image includes two 2D images, the similarity threshold corresponding to a preset multiplier of 8 may be set to 0.2, the similarity threshold corresponding to a preset multiplier of 4 may be set to 0.4, and the similarity threshold corresponding to a preset multiplier of 2 may be set to 0.6. In some embodiments, the first preset condition may include that the first similarity degree is larger than the similarity threshold, etc.

In some embodiments, when the first similarity degree is larger than the similarity threshold, it may be determined that the at least one first projection image and the at least one downsampled 2D image satisfy the first preset condition. When it is determined that the at least one first projection image and the at least one downsampled 2D image satisfy the first preset condition, the process 400 may proceed to operation 460. If the at least one first projection image and the at least one downsampled 2D image do not satisfy the first preset condition, operations 420 to 440 may be repeated until the first preset condition is satisfied. That is, if the first similarity degree is not larger than the similarity threshold, the process 400 may return to perform operation 420 to adjust the posture corresponding to the downsampled 3D image based on the preset step size. Operation 430 may be performed to obtain the first projection image corresponding to each 2D image.

Operation 440 may be performed to determine the first similarity degree based on the at least one first projection image and the at least one downsampled 2D image. A process of operations 420 to 440 may be repeated until the first similarity degree is larger than the similarity threshold.

In 460, in response to determining that the at least one first projection image and the at least one downsampled 2D image satisfy the first preset condition, the registered 3D image may be obtained by upsampling the adjusted downsampled 3D image based on the preset multiplier. The upsampling operation may enlarge the adjusted downsampled 3D image, so that the registered 3D image has the same size as the original 3D image.

In some embodiments, after it is determined that the at least one first projection image and the at least one downsampled 2D image satisfy the first preset condition, the posture transformation module 220 may further determine whether the preset multiplier satisfies a second preset condition. If the preset multiplier satisfies the second preset condition, the posture transformation module 220 may upsample the adjusted downsampled 3D image, and designate the upsampled image as the registered 3D image. For example, the second preset condition may include that the preset multiplier is equal to a preset value of a multiplier. The preset value of the multiplier may be a preset relatively small value. If the preset multiplier is larger than the preset value, it may indicate that the preset multiplier is relatively large, and a size difference between the downsampled 3D image and the 3D image is relatively large. Then, the preset multiplier needs to be decreased based on a preset downsampling step size to reduce the size difference between the downsampled 3D image and the 3D image. By gradually decreasing the preset multiplier and continuously adjusting the posture corresponding to the downsampled 3D image at each preset multiplier, a multi-level brute force search may be implemented, thereby improving the speed of the rough registration.

In some embodiments, if the preset multiplier does not satisfy the second preset condition, that is, the preset multiplier is not equal to the preset value (e.g., the preset multiplier is larger than the preset value), the adjusted downsampled 3D image may be upsampled to obtain an updated 3D image. A posture corresponding to the updated 3D image may be different from the posture corresponding to the original 3D image. Furthermore, the preset multiplier may be decreased, and operations 410 to 450 may be repeated. That is, the updated 3D image and the 2D image corresponding to the updated 3D image may be downsampled based on the decreased preset multiplier, and the process of the obtaining the first projection image corresponding to each of the at least one 2D image may be repeated until the at least one first projection image and the at least one downsampled 2D image corresponding to the at least one first projection image satisfy the first preset condition and the preset multiplier satisfies the second preset condition.

In some embodiments, the preset multiplier may be reduced according to the preset downsampling step size. For instance, the decreased preset multiplier may be obtained by dividing the preset multiplier by the downsampling step size. The downsampling step size may be set according to actual requirement(s). For example, if the preset downsampling step size is 2, and the downsampling is performed on the preset multiplier based on the downsampling step size, the preset multiplier may be decreased by 2 times.

Since the adjusted downsampled 3D image already satisfies the first preset condition, continuing to adjust the posture based on the adjusted downsampled 3D image can reduce a duration of the rough registration. The updated 3D image may be obtained by restoring (i.e., upsampling) the adjusted downsampled 3D image to the same size as the original 3D image based on the preset multiplier, facilitating subsequent downsampling on the updated 3D image based on the decreased preset multiplier, thereby reducing the size difference between the downsampled 3D image and the 3D image.

In some embodiments, spatial transformation parameters (e.g., the preset multiplier and the preset step size) may be adjusted using a global optimizer. Optimization problems include various types of problems, such as resource allocation for maximum efficiency, fitting problems, minimization-maximization problems, etc. The optimization problems are generally divided into local optimization problems and global optimization problems. A local optimization problem refers to determining a minimum value within a finite region in a function value space, and a global optimization problem refers to determining a minimum value in the entire region of the function value space. A local minimum point in a function is a point whose function value is less than or equal to values of nearby points but may be larger than values of points at a greater distance. A global minimum point is a function point whose function value is less than or equal to values of all feasible points.

In some embodiments, when the first similarity degree does not satisfy the preset threshold, the posture transformation module 220 may adjust spatial transformation parameter(s) (e.g., the preset multiplier, the preset step size, etc.) using a global optimization manner to quickly and accurately determine the spatial transformation parameter making that the first similarity degree satisfy the first preset threshold, which improves the efficiency.

In some other embodiments, the posture transformation module 220 may adjust the spatial transformation parameter(s) (e.g., the preset multiplier, the preset step size, etc.) in other optimization manners, which is not limited herein.

In 470, the first transformation matrix may be determined based on the posture transformation process from the 3D image to the registered 3D image.

In some embodiments, the posture transformation module 220 may obtain a second transformation matrix between a 2D imaging device coordinate system and the surgical space coordinate system. The 2D imaging device coordinate system is a coordinate system generated with a medical imaging device (e.g., a 2D medical imaging device) during a surgery as a reference object. For example, the 2D imaging device coordinate system may be established with a specific position (e.g., a point on a C-arm) on the 2D imaging device as a coordinate origin, a horizontal direction as an x-axis, a vertical direction as a z-axis, and a front-rear direction as a y-axis.

For instance, during or before the surgery, a first tracking array and a second tracking array may be pre-placed on the 2D medical imaging device (e.g., a C-arm of a DR device) and the body of a target object, respectively. The first tracking array and the second tracking array may include a plurality of markers (e.g., retroreflective spheres). A device space coordinate system (i.e., the 2D imaging device coordinate system) corresponding to the 2D medical imaging device may be established based on the first tracking array, and an intraoperative space coordinate system (i.e., the surgical space coordinate system) may be established based on the second tracking array. Coordinates of the first tracking array and the second tracking array may be obtained using an OTS, thus the transformation matrix between the 2D imaging device coordinate system and the surgical space coordinate system may be determined as the second transformation matrix.

Furthermore, the posture transformation module 220 may obtain a third transformation matrix between the 3D image coordinate system and the 2D imaging device coordinate system based on the registered 3D image. The process of adjusting the 3D image to obtain the registered 3D image is a process of establishing a transformation relationship between the 3D image coordinate system and the 2D imaging device coordinate system. Therefore, a first adjustment value may be determined based on the adjustment process, and the third transformation matrix may be determined based on the first adjustment value. The first adjustment value may include a total rotation step size and a total translation step size. For example, operation 420 may be performed multiple times. The posture transformation module 220 may obtain the total rotation step size by adding the rotation step size used in each execution of operation 420, and obtain the total translation step size by adding the translation step size used in each execution of operation 420.

The posture transformation module 220 may obtain the first transformation matrix based on the second transformation matrix and the third transformation matrix. For example, a product of the second transformation matrix and the third transformation matrix may be designated as the first transformation matrix.

For purposes of illustration, a specific example of obtaining the registered 3D image by adjusting the posture corresponding to the 3D image is provided below. Assuming that the at least one 2D image includes two 2D images, the preset value for the multiplier is 2, the preset downsampling step size is 2, the similarity threshold for the preset multiplier of 8 is 0.2, the similarity threshold for the preset multiplier of 4 is 0.4, and the similarity threshold for the preset multiplier of 2 is 0.6, the posture transformation module 220 may obtain the registered 3D image and determine the third transformation matrix through the following operations.

A downsampled 3D image P02, a downsampled 2D image P12, and a downsampled 2D image P22 may be obtained by performing a downsampling operation with a preset multiplier of 8 on a 3D image P01, a 2D image P11 corresponding to a first capturing posture, and a 2D image P21 corresponding to a second capturing posture, respectively. A first projection image D1 and a first projection image D2 may be obtained by projecting the downsampled 3D image P02 at a first projection posture and a second projection posture, respectively. The first capturing posture is the same as the first projection posture, and the second capturing posture is the same as the second projection posture. A first similarity degree may be determined based on the downsampled 2D image P12, the downsampled 2D image P22, the first projection image D1, and the first projection image D2. If the first similarity degree is not larger than the similarity threshold of 0.2, the posture corresponding to the downsampled 3D image P02 may be adjusted with a preset rotation step of 10 degrees and a preset translation step of 20 millimeters corresponding to the preset multiplier of 8, and an adjusted downsampled 3D image P02 may be obtained. The process of the determining the first similarity degree may be repeated until the determined first similarity degree is larger than 0.2. Furthermore, whether a current preset multiplier is equal to the preset value of 2 may be determined. Since the preset multiplier of 8 is not equal to the preset value of 2, the adjusted downsampled 3D image P02 may be upsampled according to the preset multiplier of 8, such that the 3D image P01 may be updated to obtain a 3D image P01'. The preset multiplier of 8 may be decreased to 4 based on the preset downsampling step size of 2.

A downsampled 3D image P02', a downsampled 2D image P12', and a downsampled 2D image P22' may be obtained by performing a downsampling processing with a preset multiplier of 4 on the 3D image P01', the 2D image P11 corresponding to the first capturing posture, and the 2D image P21 corresponding to the second capturing posture, respectively. A first projection image D1' at the first projection posture and a first projection image D2' at the second projection posture may be obtained by projecting the downsampled 3D image P02' at the first projection posture and the second projection posture, respectively. A first similarity degree may be determined based on the downsampled 2D image P12', the downsampled 2D image P22', the first projection image D1' at the first projection posture, and the first projection image D2' at the second projection posture. If the first similarity degree is not larger than 0.4, the posture corresponding to the downsampled 3D image P02' may be adjusted with a preset rotation step of 5 degrees and a preset translation step of 10 millimeters corresponding to the preset multiplier of 4, and an adjusted downsampled 3D image P02' may be obtained. The process of the determining the first similarity degree may be repeated until the determined first similarity degree is larger than 0.4. Furthermore, whether a current preset multiplier is equal to the preset value of 2 may be determined. Since the preset multiplier of 4 is not equal to the preset value of 2 in the operation, the adjusted downsampled 3D image P02' may be upsampled according to the preset multiplier of 4, such that the 3D image P01 may be updated to obtain a 3D image P01". The preset multiplier of 4 may be decreased to 2 according to the preset downsampling step size of 2.

A downsampled 3D image P02", a downsampled 2D image P12", and a downsampled 2D image P22" may be obtained by performing a downsampling processing with a preset multiplier of 2 on the 3D image P01", the 2D image P11 corresponding to the first projection posture, and the 2D image P21 corresponding to the second projection posture, respectively. A first projection image D1" at the first projection posture and a first projection image D2" at the second projection posture may be obtained by projecting the downsampled 3D image P02" at the first projection posture and the second projection posture, respectively. A first similarity degree may be determined based on the downsampled 2D image P12", the downsampled 2D image P22", the first projection image D1" at the first projection posture, and the first projection image D2" at the second projection posture. If the first similarity degree is not larger than 0.6, the posture corresponding to the downsampled 3D image P02" may be adjusted with a preset rotation step of 5 degrees and a preset translation step of 10 millimeters corresponding to the preset multiplier of 2, and an adjusted downsampled 3D image P02" may be obtained. The process of the determining the first similarity degree may be repeated until the determined first similarity degree is larger than 0.6. Furthermore, whether a current preset multiplier is equal to the preset value of 2 may be determined. Since the preset multiplier of 2 is equal to the preset value of 2 in the operation, the adjusted downsampled 3D image P02" may be upsampled based on the preset multiplier of 2, such that the adjusted 3D image may be obtained and the third transformation matrix may be determined.

According to some embodiments of the present disclosure, the rough registration between the 3D image and the at least one 2D image can be achieved by performing the posture transformation on the 3D image based on the at least one 2D image. In the rough registration process, no anatomical points need to be selected manually in the 3D image and the at least one 2D image, and no large amount of human-computer interaction operations need to be performed, which reduces the complexity of the registration. Furthermore, by downsampling the 3D image and the at least one 2D image, and analyzing based on the downsampled images, an amount of data analysis can be reduced and the speed of the rough registration can be improved, such that rapid and automatic rough registration between the 3D image and the at least one 2D image can be achieved.

FIG. 5 is a flowchart illustrating an exemplary process for generating a 3D target image and at least one 2D target image according to some embodiments of the present disclosure.

In some embodiments, the target image determination module 230 may determine a 2D target image corresponding to a target part of a target object in each of at least one 2D image and determine a 3D target image corresponding to the target part in a registered 3D image, respectively, through a manner as shown in a process 500. The process 500 may be performed to achieve at least part of operation 330 as described in connection with FIG. 3. In some embodiments, when the target object includes a plurality of target parts, the process 500 may be executed respectively for each of the plurality of target parts. As shown in FIG. 5, the process 500 may include the following operations.

In 510, the 3D target image corresponding to the target part may be determined in the registered 3D image. The 3D target image refers to an image that includes only the target part or includes the target part and a small amount of surrounding regions of the target part extracted from the registered 3D image. For example, the target part may include at least one of a target spine, a target hip joint, a target elbow joint, a target knee joint, a target finger joint, or the like.

In some embodiments, the target image determination module 230 may obtain a segmentation mask corresponding to the target part by segmenting the registered 3D image via an image segmentation algorithm. The target image determination module 230 may determine the 3D target image that includes only the target part in the registered 3D image based on the segmentation mask.

In some embodiments, after operation 510 is performed, for each of the at least one 2D image, a fourth transformation matrix between a 2D image coordinate system corresponding to the 2D image and a surgical space coordinate system may be obtained via operation 520, and the 2D target image may be determined based on the 3D target image, a first transformation matrix, and the fourth transformation matrix via operations 530 to 550. For ease of illustration, the implementation process of operations 520 to 550 is described below by taking a single 2D image as an example.

In 520, the fourth transformation matrix between the 2D image coordinate system corresponding to the 2D image and the surgical space coordinate system may be obtained.

In some embodiments, the target image determination module 230 may obtain a second transformation matrix between a 2D imaging device coordinate system and the surgical space coordinate system. More descriptions regarding the obtaining the second transformation matrix may be found in operation 470 and relevant descriptions thereof. The target image determination module 230 may also obtain a fifth transformation matrix between the 2D imaging device coordinate system and the 2D image coordinate system. For instance, the target image determination module 230 may obtain intrinsic parameter(s) (e.g., a distance from a radiation source to an imaging plane) of a 2D medical imaging device when the 2D medical imaging device captures the 2D image, and determine the fifth transformation matrix based on the intrinsic parameter(s). The target image determination module 230 may obtain the fourth transformation matrix between the 2D image coordinate system corresponding to the 2D image and the surgical space coordinate system based on the second transformation matrix and the fifth transformation matrix.

In 530, at least one 3D coordinate of at least one representative point of the target part in the 3D image coordinate system and a size parameter of the target part may be determined based on the 3D target image.

A representative point of the target part refers to a representative characteristic point in the target part, such as a center point, a boundary point, etc. In some embodiments, the representative point of the target part may include the center point of the target part, such as the center of the spine.

The size parameter of the target part refers to a parameter related to a size of the target part, such as a length of the target part, a width of the target part, a distance from the center point of the target part to an edge of the target part, etc. In some embodiments, the size parameter of the target part may include a collection of distances from the center point of the target part to edges of the target part. Optionally, the edges of the target part may be represented by various edges of a rectangle enclosing the target part.

In some embodiments, the target image determination module 230 may identify the at least one representative point (e.g., the center point) of the target part in the 3D target image, thereby obtaining the at least one 3D coordinate of the at least one representative point of the target part in the 3D coordinate system.

In some embodiments, the target image determination module 230 may obtain a second projection image corresponding to the 2D image by projecting (e.g., DRR projection) the 3D target image. A projection posture of the second projection image corresponding to the 2D image is the same as a capturing posture of the 2D image. In some embodiments, projection images (e.g., the second projection image, etc.) corresponding to the 2D images may be obtained using a manner similar to the manner for obtaining the first projection image. The target image determination module 230 may determine the at least one representative point and the size parameter of the target part in the second projection image based on the representative point of the target part in the 3D target image. For example, the target image determination module 230 may determine a center point (i.e., a second center point) in the second projection image by projecting a center point (i.e., a first center point) of the 3D target image, and determine a minimal enclosing rectangle of the target part in the second projection image so as to obtain distances from the second center point to each edge of the minimal enclosing rectangle. A distance collection formed by the distances may be designated as the size parameter of the target part. That is, distances from the second center point (i.e., the center point of the target part in the second projection image) to the edges of the target part refer to the distances from the second center point to each edge of the minimal enclosing rectangle of the target part.

Merely by way of example, the at least one 2D image may include a first 2D image and a second 2D image. The target image determination module 230 may obtain a second projection image X1 corresponding to the first 2D image and a second projection image X2 corresponding to the second 2D image by projecting the 3D target image with a first projection posture and a second projection posture, respectively. A second center point f1 may be determined by projecting the first center point onto the second projection image X1, and a second center point f2 may be determined by projecting the first center point onto the second projection image X2. A minimal enclosing rectangle C1 of the target part may be determined in the second projection image X1, distances from the second center point f1 to each edge of the minimal enclosing rectangle C1 may be determined, and a distance collection A1 corresponding to the second projection image X1 may be obtained. Similarly, a minimal enclosing rectangle C2 of the target part may be determined in the second projection image X2, distances from the second center point f2 to each edge of the minimal enclosing rectangle C2 may be determined, and a distance collection A2 corresponding to the second projection image X2 may be obtained. The distance collection A1 may be designated as the size parameter corresponding to the first 2D image, and the distance collection A2 may designated as the size parameter corresponding to the second 2D image.

In 540, at least one 2D coordinate of the at least one representative point in the 2D image coordinate system may be determined based on the at least one 3D coordinate, the first transformation matrix, and the fourth transformation matrix. The 3D coordinate may be determined through operation 530, the fourth transformation matrix may be determined through operation 520, and the first transformation matrix may be determined through operation 320.

In some embodiments, the target image determination module 230 may obtain at least one first transformation point by transforming the at least one 3D coordinate of the at least one representative point in the 3D image coordinate system to the surgical space coordinate system using the first transformation matrix. Further, the target image determination module 230 may obtain the at least one 2D coordinate of the at least one representative point by transforming the at least one first transformation point in the surgical space coordinate system to the 2D image coordinate system corresponding to the 2D image using the fourth transformation matrix corresponding to the 2D image.

Merely by way of example, the target image determination module 230 may obtain a first transformation point m2 by transforming a 3D center point m1 to the surgical space coordinate system using a first transformation matrix T1. Then, the target image determination module 230 may obtain a 2D center point n1 (i.e., a corresponding point of the representative point in the first 2D image) by transforming the first transformation point m2 to a first 2D image coordinate system (i.e., the coordinate system corresponding to the first 2D image) using a fourth transformation matrix T4a corresponding to the first 2D image. Similarly, the target image determination module 230 may obtain a 2D center point n2 (i.e., a corresponding point of the representative point in the second 2D image) by transforming the first transformation point m2 to a second 2D image coordinate system (i.e., the coordinate system corresponding to the second 2D image) using a first transformation matrix T4b corresponding to the second 2D image.

In 550, the 2D target image may be determined based on the at least one 2D coordinate and the size parameter.

For example, as described in operation 530, the size parameter may include the distance collection formed by the distances from the center point of the target part to the edges of the minimal enclosing rectangle. The target image determination module 230 may use the 2D coordinate as the center and determine an extracted region based on the distance collection. The extracted region may be designated as the 2D target image. Merely by way of example, the 2D target image may be determined in the first 2D image based on the second center point f1 and the distance collection A1 corresponding to the first 2D image. The 2D target image may be determined in the second 2D image based on the second center point f2 and the distance collection A2 corresponding to the second 2D image.

In some embodiments, a distance from the center point to an edge of the minimal enclosing rectangle may be represented by a count of pixels. In some embodiments, the distance collection may include a left distance, a right distance, a top distance, and a bottom distance. In some embodiments, the target image determination module 230 may determine a coordinate of an upper-left point based on a 2D coordinate of the representative point in the 2D image coordinate system, the left distance, and the top distance; determine a coordinate of an upper-right point based on the 2D coordinate of the representative point, the right distance, and the top distance; determine a coordinate of a lower-left point based on the 2D coordinate of the representative point, the left distance, and the bottom distance; and determine a coordinate of a lower-right point based on the 2D coordinate of the representative point, the right distance, and the bottom distance. Then, the extracted region may be determined based on the coordinates of the upper-left point, the upper-right point, the lower-left point, and the lower-right point.

In some embodiments, considering errors from the rough registration, the target image determination module 230 may extend the distance collection, so that the 2D target image includes the entire target part. For example, the target image determination module 230 may add a preset count of pixels to the left distance, the right distance, the top distance, and the bottom distance, respectively, such as adding 40 pixels to each of the left distance, the right distance, the top distance, and the bottom distance.

In some embodiments, the 2D target image may be obtained by directly segmenting the 2D image through a segmentation algorithm rather than relying on the 3D image.

Since the 2D image usually has a lower imaging definition than the 3D image and may include surgical device(s), an optimal segmentation result can not be obtained by directly segmenting the target spine from the 2D image. According to some embodiments of the present disclosure, by determining the 2D target image based on the 3D target image in the registered 3D image, the segmentation accuracy can be improved.

FIG. 6 is a flowchart illustrating an exemplary process for obtaining a target transformation matrix by optimizing a first transformation matrix according to some embodiments of the present disclosure.

In some embodiments, the target matrix determination module 240 may obtain a target transformation matrix by performing posture transformation on a registered 3D image based on a 3D target image and a 2D target image in each of at least one 2D image to optimize a first transformation matrix through a manner as shown in a process 600. The process 600 may be performed to achieve at least part of operation 340 as described in connection with FIG. 3. In some embodiments, the process 600 may be performed by the target matrix determination module 240. As shown in FIG. 6, the process 600 may include the following operations.

In 610, for each of the at least one 2D image, a second projection image corresponding to the 2D image may be obtained by projecting the 3D target image based on a capturing posture of the 2D image.

The second projection image may be obtained in a similar manner as how the first projection image is obtained in operation 430, which is not repeated herein.

In 620, a second similarity degree may be determined based on the at least one second projection image and the at least one 2D target image.

A second similarity degree may be used to determine a similarity degree between a 2D target image and a second projection image corresponding to the 2D target image.

In some embodiments, similar to the determination of the first similarity degree, the target matrix determination module 240 may determine a similarity degree between each of the at least one 2D target image and the second projection image corresponding to the 2D target image using various techniques (e.g., mutual information, pattern intensity, gradient difference, etc.), and then obtain the second similarity degree based on all determined similarity degrees. The various techniques may include summation, averaging, obtaining a maximum value, etc. More descriptions regarding the determination of the similarity degree may be found in operation 440 and relevant descriptions thereof.

In 630, the target transformation matrix may be obtained by adjusting a posture corresponding to the registered 3D image based on the second similarity degree to optimize the first transformation matrix.

In some embodiments, the target matrix determination module 240 may obtain the target transformation matrix by adjusting the posture corresponding to the registered 3D image in a similar manner as how the registered 3D image is obtained. For example, the target matrix determination module 240 may perform operations similar to operations 450-470, wherein the registered 3D image is equivalent to the original 3D image in operations 450-470, the target transformation matrix is equivalent to the first transformation matrix in operations 450-470, and the second similarity degree is equivalent to the first similarity degree in operations 450-470.

In some embodiments, the target matrix determination module 240 may determine whether the second similarity degree satisfies a third preset condition. For example, the third preset condition may include that a similarity difference between the second similarity degree and a last second similarity degree determined during a last adjustment of the posture corresponding to the registered 3D image is less than a difference threshold. The threshold may be set according to requirement(s), for example, 0.005, etc. As another example, the third preset condition may include that the second similarity degree is larger than a similarity threshold.

In some embodiments, the target matrix determination module 240 may determine whether a current iteration count satisfies a fourth preset condition. For example, the fourth preset condition may be that the current iteration count is equal to a preset iteration count. The current iteration count is a count of iterations that the posture corresponding to the registered 3D image is adjusted, and the preset iteration count may be set according to requirement(s), for example, 40 iterations.

In some embodiments, if the second similarity degree does not satisfy the third preset condition and/or the current iteration count does not satisfy the fourth preset condition, the target matrix determination module 240 may adjust the posture corresponding to the registered 3D image based on the second similarity degree to optimize the first transformation matrix. The process of the determining the second similarity degree may be repeated until the determined second similarity degree satisfies the third preset condition and/or the current iteration count satisfies the fourth preset condition. The optimized first transformation matrix may be determined as the target transformation matrix.

In some embodiments, an optimizer (e.g., a Powell optimization algorithm, etc.) may be used to adjust the posture corresponding to the registered 3D image. When the posture corresponding to the registered 3D image is optimal, the optimization process may be completed, and the first transformation matrix obtained at the completion of the optimization process may be determined as the target transformation matrix. In some embodiments, whether the posture corresponding to the registered 3D image is optimal may be determined based on whether the optimizer meets an iteration stop condition, such as, whether a maximum count of iterations (e.g., 40, etc.) is reached, a minimum step size (e.g., 0.05, etc.) is reached, an allowable change (e.g., 0.005) in step size is reached, etc.

Merely by way of example, the target matrix determination module 240 may iteratively adjust the posture corresponding to the registered 3D image based on a plurality of 2D target images to optimize the first transformation matrix until the similarity difference between the currently determined second similarity degree and the last second similarity degree determined during the last adjustment of the posture corresponding to the registered 3D image is less than a threshold (e.g., 0.005), or until the current iteration count is equal to the preset iteration count (e.g., 40). After adjusting the posture corresponding to the registered 3D image for an i-th time, a plurality of second projection images may be determined based on the adjusted registered 3D image. A second similarity degree Si may be determined based on the plurality of second projection images and the plurality of 2D target images. A similarity difference between the second similarity degree Si and Si-1 (i.e., a second similarity degree obtained after adjusting the posture corresponding to the registered 3D image for the i-1-th time) may be determined, and the current iteration count i may be obtained. If the similarity difference is not less than a threshold, and i is not equal to the preset iteration count, the posture corresponding to the adjusted registered 3D image may be further adjusted based on the second similarity degree Si.

In some embodiments, the target matrix determination module 240 may obtain the similarity difference between the second similarity degree and the last second similarity degree determined during the last adjustment of the posture corresponding to the registered 3D image. The target matrix determination module 240 may determine an adjustment step size using the similarity difference, and adjust the posture corresponding to the adjusted registered 3D image again. The adjustment step size may include an adjustment rotation step size and an adjustment translation step size. The similarity difference and the adjustment step size may be positively correlated. That is, the larger the similarity difference, the larger the adjustment step size. By defining the positive correlation between the similarity difference and the adjustment step size, the posture corresponding to the adjusted registered 3D image may be adaptively adjusted based on the similarity difference, which can accelerate the speed of iterative optimization and improve the accuracy of the target transformation matrix.

In some embodiments, if a difference between the adjustment step size and a last adjustment step size determined during the last adjustment of the posture of the registered 3D image is less than a difference threshold, the target transformation matrix may be obtained. The difference threshold may be set according to requirement(s), for example, as 0.005, etc.

During the adjustment process of the posture corresponding to the registered 3D image based on the 3D target image and the 2D target image of each 2D image, multiple posture adjustment are performed on the registered 3D image based on the adjustment step size, and a total adjustment step size during the process may be determined. The total adjustment step size may include a total adjustment rotation step size and a total adjustment translation step size. The target matrix determination module 240 may determine the target transformation matrix based on the first transformation matrix, the total adjustment rotation step size, and the total adjustment translation step size. The target transformation matrix may be used to achieve a fine registration of the target part in a 3D image coordinate system with an intraoperative space coordinate system.

According to the embodiments of the present disclosure, during the fine registration process, no other human-computer interaction is required besides determining the target part, which simplify the process. The second similarity degree can be determined based on the at least one second projection image and the at least one 2D target image, and then the adaptive iterative adjustment of the posture corresponding to the registered 3D image can be performed based on the second similarity degree, such that the first transformation matrix can be iteratively optimized until the target transformation matrix is obtained. In this way, the speed of iterative optimization of the first transformation matrix can be accelerated, and the accuracy of the target transformation matrix can be improved.

It should be noted that the descriptions of processes 300, 400, 500, and 600 are merely provided for the purposes of illustration and explanation, and are not intended to limit the scope of the present disclosure. For persons having ordinary skills in the art, various variations and modifications to processes 300, 400, 500, and 600 may be conducted under the guidance of the present disclosure. However, these variations and modifications may not depart from the scope of the present disclosure. For example, the order of operations 520 and 530 may be exchanged.

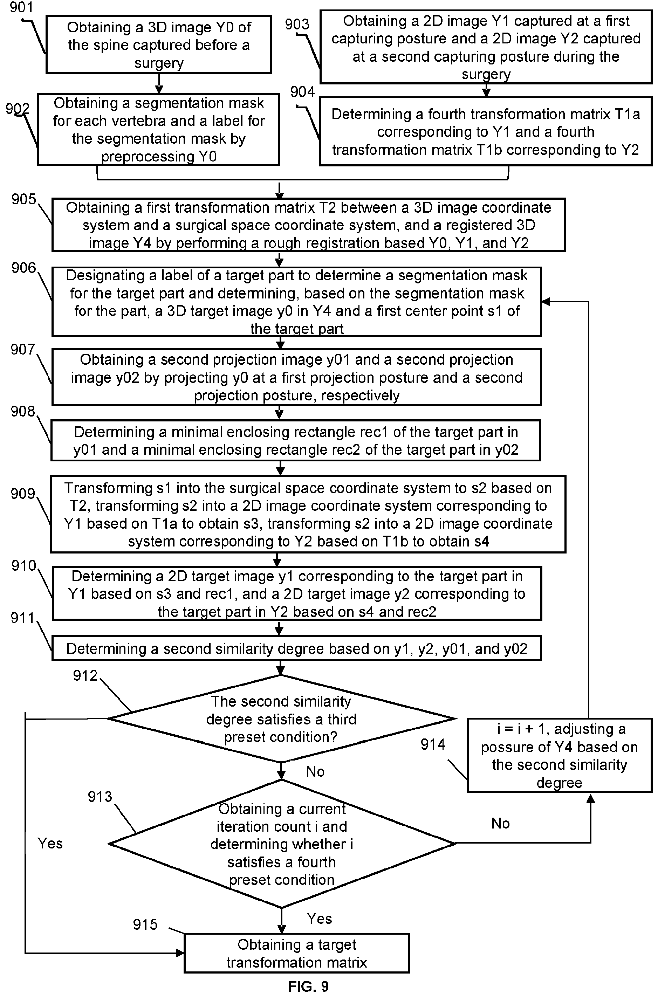

FIG. 9 is a schematic diagram illustrating a registration process according to some embodiments of the present disclosure.

In some embodiments, the registration process illustrated in FIG. 9 may be performed by the processing device 120. In some embodiments, a target part may be a vertebra in a spine. The registration process illustrated in FIG. 9 may include the following operations.

In 901, a 3D image Y0 of the spine captured before a surgery may be obtained.

In 902, a segmentation mask of each vertebra in the spine and a label of the segmentation mask of each vertebra may be obtained by preprocessing the 3D image Y0. For example, the segmentation mask for each vertebra may be obtained by segmenting the vertebrae in the 3D image Y0 through an image segmentation algorithm, and then each segmentation mask may be labeled.

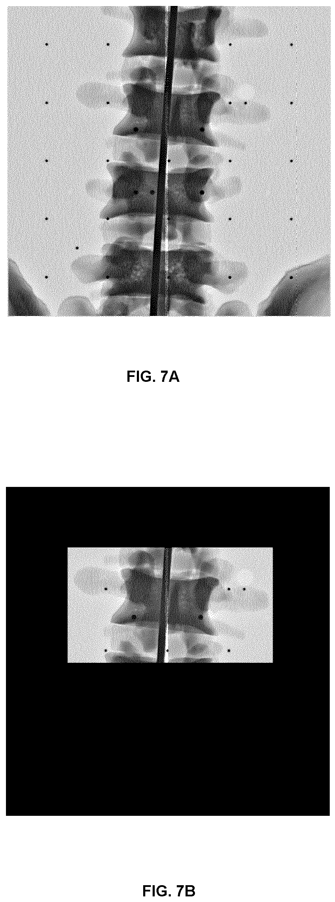

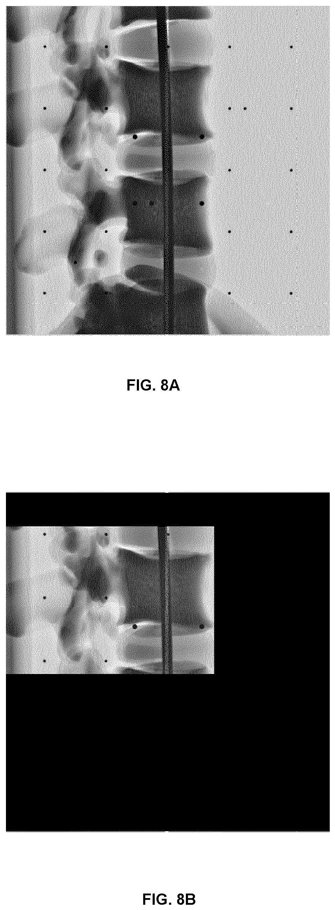

In 903, a 2D image Y1 of the spine captured at a first capturing posture during the surgery and a 2D image Y2 of the spine captured at a second capturing posture during the surgery may be obtained. For example, the 2D image Y1 may include a 2D image (as shown in FIG. 7A) of the spine captured at an anteroposterior position, and the 2D image Y2 may include a 2D image (as shown in FIG. 8A) of the spine captured at a lateral position.

In 904, a fourth transformation matrix T1a corresponding to the 2D image Y1 and a fourth transformation matrix T1b corresponding to the 2D image Y2 may be determined based on the 2D images Y1 and Y2.

In 905, a first transformation matrix T2 between a 3D image coordinate system and a surgical space coordinate system and a registered 3D image Y4 may be obtained by performing a rough registration based on the 3D image Y0, the 2D image Y1, and the 2D image Y2.

In 906, the label of the target part may be designated to determine the segmentation mask of the target part. A 3D target image y0 in the registered 3D image Y4 and a first center point s1 of the target part may be determined based on the segmentation mask of the target part.

In some embodiments, the processing device 120 may determine a 2D target image that only includes the target part from the 2D images by performing operations 907 to 910.

In 907, a second projection image y01 corresponding to a first projection posture and a second projection image y02 corresponding to a second projection posture may be obtained by projecting the 3D target image y0 at the first projection posture and the second projection posture, respectively. The first projection posture is the same as the first capturing posture, and the second projection posture is the same as the second capturing posture.

In 908, a minimal enclosing rectangle rec1 of the target part in the second projection image y01 and a minimal enclosing rectangle rec2 of the target part in the second projection image y02 may be determined.

In 909, a transformation point s2 may be obtained by transforming the center point s1 into the surgical space coordinate system based on the first transformation matrix T2. A transformation point s3 may be obtained by transforming the transformation point s2 into a 2D image coordinate system corresponding to the 2D image Y1 based on the fourth transformation matrix T1a. A transformation point s4 may be obtained by transforming the transformed point s2 into the 2D image coordinate system corresponding to the 2D image Y2 based on the fourth transformation matrix T1b.

In 910, a 2D target image y1 corresponding to the target part in the 2D image Y1 may be determined based on the transformation point s3 and the minimal enclosing rectangle rec1. A 2D target image y2 corresponding to the target part in the 2D image Y2 may be determined based on the transformation point s4 and the minimal enclosing rectangle rec2. For example, the 2D target image y1 may include an anteroposterior 2D image of the target vertebra as shown in FIG. 7B, which is segmented from the anteroposterior 2D image of the target vertebra as shown in FIG. 7A. The 2D target image y2 may include a lateral 2D image of the target vertebra as shown in FIG. 8B, which is segmented from the lateral 2D image of the target vertebra as shown in FIG. 8A.

In some embodiments, the processing device 120 may obtain a target transformation matrix by performing a fine registration process, i.e., performing posture transformation on the registered 3D image based on the 3D target image and the 2D target image in each of at least one 2D image to optimize the first transformation matrix.

In 911, a second similarity degree may be determined based on the 2D target image y1, the 2D target image y2, the second projection image y01, and the second projection image y02.

In 912, whether the second similarity degree satisfies a third preset condition may be determined. If the second similarity degree does not satisfy the third preset condition, operation 913 may be performed. If the second similarity degree satisfies the third preset condition, operation 915 may be performed. The third preset condition may include that a similarity difference between a currently determined second similarity degree and a last second similarity degree determined during a last adjustment of the posture corresponding to the registered 3D image Y4 is less than a threshold.

In 913, a current iteration count i may be obtained and whether the current iteration count i satisfies a fourth preset condition may be determined. If the current iteration count i does not satisfy the fourth preset condition, operation 914 may be performed. If the current iteration count i satisfies the fourth preset condition, operation 915 may be performed. The fourth preset condition may include that the current iteration count i is equal to a preset iteration count.

In 914, the current iteration count i may be increased by 1, the posture corresponding to the registered 3D image Y4 may be adjusted based on the second similarity degree, and operation 906 may be repeated.

In 915, the target transformation matrix may be obtained.

It should be understood that the registration process shown in FIG. 9 is exemplary, which may include one or more additional operations or omit one or more of the above operations. For example, operation 914 may be omitted. If the second similarity degree threshold does not satisfy the third preset condition, operation 915 may be performed.

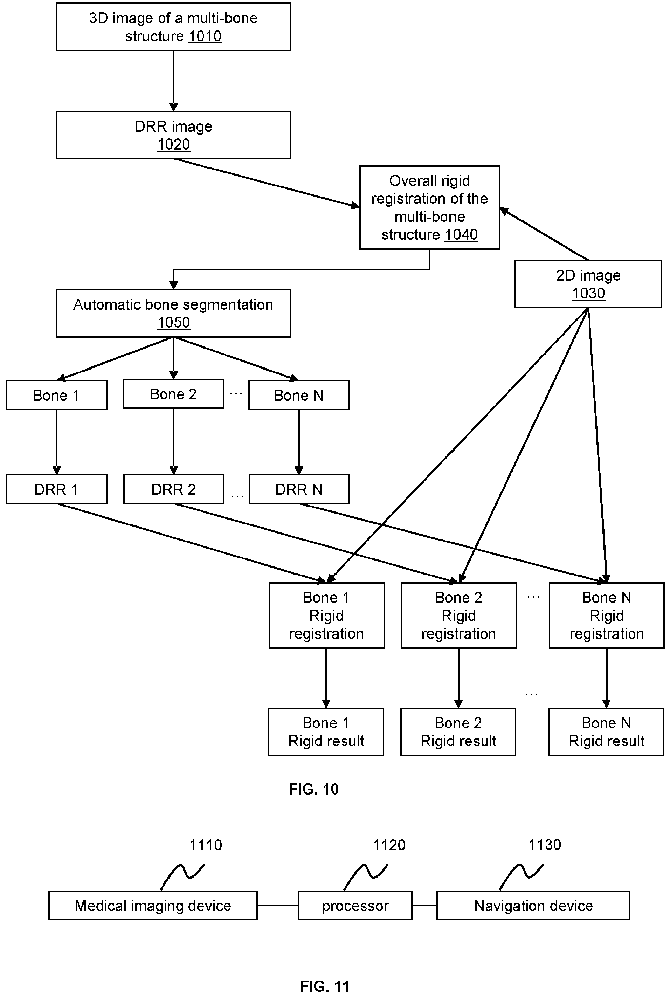

FIG. 10 is a schematic diagram illustrating a registration process according to some embodiments of the present disclosure.

In some embodiments, the registration process shown in FIG. 10 may be performed by the processing device 120 to register each bone in a multi-bone structure. A target part may be each bone in the multi-bone structure.

In some embodiments, the processing device 120 may obtain a 3D image to be registered and a plurality of 2D images to be registered of the multi-bone structure. As shown in FIG. 10, the processing device 120 may obtain a DRR image 1020 by performing DRR projection on a 3D image 1010 of the multi-bone structure. The 3D image 1010 of the multi-bone structure may include one of a CT image, an MRI image, etc. In some embodiments, the 3D image 1010 of the multi-bone structure may be an image including a plurality of target parts (e.g., a spine with a plurality of vertebrae).

In some embodiments, the processing device 120 may obtain the 3D image through the following operations. A preoperative original 3D image may be obtained. A first 3D image may be obtained by performing a posture search on the preoperative original 3D image based on a first posture search parameter, and a first posture similarity between the first 3D image and a 2D image may be obtained. If the first posture similarity satisfies a third preset threshold, a second 3D image may be obtained by performing a posture search on the first 3D image based on a second posture search parameter, and a second posture similarity between the second 3D image and the 2D image may be obtained. If the second posture similarity satisfies a fourth preset threshold, a 3D image may be obtained based on the original 3D image, the first posture search parameter, and the second posture search parameter, and a spatial transformation parameter for the transformation of the preoperative original 3D image to the 3D image may be determined as a first spatial transformation parameter. The first 3D image may be projected based on a capturing posture of the 2D image, and a similarity between a projection of the first 3D image and the 2D image may be determined as the first posture similarity. The second 3D image may be projected based on the capturing posture of the 2D image, and a similarity between a projection of the second 3D image and the 2D image may be determined as the second posture similarity. The posture search parameter(s) may include a preset multiplier for downsampling, a preset rotation step size, and a preset translation step size. In some embodiments, the posture search may be performed in a similar manner as operations 410 and 420. In some embodiments, other techniques for the posture search may be used based on actual requirement(s), which is not limited in the present disclosure.

Merely by way of example, the processing device 120 may estimate an initial posture of a joint part in the original 3D image using a multi-resolution multi-level search manner. Firstly, a first-level posture search may be performed by downsampling the original 3D image by a factor of 8, rotating the original 3D image around X, Y, and Z axes at intervals of 10 degrees, and translating the original 3D image along the X, Y, and Z axes at intervals of 20 millimeters in a 3D space. Then, the first posture similarity may be determined. If the first posture similarity does not satisfy a preset stop condition, the above process may be iteratively repeated until the first posture similarity satisfies the preset stop condition. A first spatial transformation parameter may be retained. A second-level posture search may be performed by downsampling the original 3D image by a factor of 4, rotating the original 3D image around the X, Y, and Z axes at intervals of 5 degrees, and translating the original 3D image along the X, Y, and Z axes at intervals of 10 millimeters. The second posture similarity may be determined. If the second posture similarity does not satisfy the preset stop condition, the above process may be iteratively repeated until the second posture similarity satisfies the preset stop condition. A second spatial transformation parameter may be retained. The initial posture of the joint part may be determined based on the first spatial transformation parameter and the second spatial transformation parameter obtained from the two-level posture search.

In some embodiments, the processing device 120 may perform a rough registration based on a DRR image of an overall 3D image and a plurality of 2D images of the multi-bone structure. That is, a similarity analysis may be performed on the DRR image and the plurality of 2D images to perform an overall rigid registration on the multi-bone structure. A projection posture of the DRR image of the overall 3D image may be the same as a capturing posture of the overall 3D image. As shown in FIG. 10, the overall rigid registration may be performed on the DRR image 1020 and the 2D image 1030 through operation 1040. For example, the 2D image 1030 may include a plurality of X-ray images.

In some embodiments, a first registered 3D image may be obtained by performing spatial transformation on the 3D image based on the first spatial transformation parameter, and a first similarity degree between the first registered 3D image and the 2D image may be obtained. In some embodiments, a plurality of first registered reconstruction images may be obtained by projecting (e.g., DRR projection) the first registered 3D image. A count of the plurality of first registered reconstruction images is the same as a count of the plurality of 2D images, and projection angles of the plurality of first registered reconstruction images are the same as capturing angles of the plurality of 2D images. In some embodiments, a similarity degree between the plurality of first registered reconstruction images and the plurality of 2D images may be determined as the first similarity degree. In some embodiments, the first registered 3D image may be projected in a similar manner as operation 430, and the first registered reconstructed image may be obtained in a similar manner as operation 440.

In some embodiments, if the first similarity degree satisfies a first preset threshold, it may be determined that the rough registration (i.e., the overall rigid registration of the multi-bone structure) is completed, and the first registered 3D image at this time may be determined as the registered 3D image of the multi-bone structure. In some embodiments, the registered 3D image of the multi-bone structure may be obtained in a similar manner as operation 460.

In some embodiments, after completing the overall rigid registration of the multi-bone structure, the processing device 120 may obtain a single-bone 3D image for each bone of the multi-bone structure based on the first registered 3D image and obtain a single-bone 2D image for each bone of the multi-bone structure based on the plurality of 2D images. That is, a plurality of single-bone images may be obtained by segmenting the image of the multi-bone structure.

In some embodiments, the processing device 120 may obtain a second registered 3D image of each bone of the multi-bone structure by performing spatial transformation on the single-bone 3D image based on the second spatial transformation parameter. In some embodiments, the second registered 3D image may be obtained in a similar manner as how the first registered 3D image is obtained.

In some embodiments, the processing device 120 may obtain a second similarity degree between the second registered 3D image and a single-bone 2D image corresponding to the second registered 3D image. If the second similarity degree satisfies a second preset threshold, a registration result may be obtained based on the second registered 3D image. In some embodiments, the second similarity degree may be obtained in a similar manner as how the first similarity degree is obtained. In some embodiments, the registration result may be obtained in a similar manner as operations 460 and 470. In some embodiments, the registration result may include a registration result of each bone of the multi-bone structure.

After completing the overall rigid registration of the multi-bone structure, the registered 3D image of the multi-bone structure may be obtained. Then, a 3D image of each bone of the multi-bone structure may be obtained by performing automatic bone segmentation on the registered 3D image of the multi-bone structure. A DRR image for each bone of the multi-bone structure may be obtained by projecting and reconstructing on the 3D image of each bone of the multi-bone structure. As shown in FIG. 10, after operation 1040, a plurality of 3D images of the bones of the multi-bone structure (i.e., Bone 1, Bone 2, ..., Bone N, wherein N may be an integer larger than or equal to 2) may be obtained by performing automatic bone segmentation on the registered 3D image of the multi-bone structure in operation 1050. Then, projection images DRR1, DRR2, ..., DRRN corresponding to the Bone 1, Bone 2, ..., Bone N may be obtained by performing DRR projection on the Bone 1, Bone 2, ..., Bone N. The projection posture of the DRR image corresponding to each bone of the multi-bone structure is the same as the capturing posture of the 2D image 1030.

In some embodiments, the processing device 120 may directly perform automatic bone segmentation on the original 3D image of the multi-bone structure (i.e., a 3D image of the multi-bone structure that has not undergone the overall rigid registration) and obtain a 3D image of each bone of the multi-bone structure. A posture corresponding to the 3D image of each bone may be adjusted based on an overall rigid registration result, and then projection and reconstruction may be performed on an adjusted 3D image of each bone to obtain the DRR image of each bone.

In some embodiments, the processing device 120 may perform fine registrations based on the DRR image of each bone and the 2D image of each bone. That is, the processing device 120 may perform a similarity degree comparison between the projection image of each bone and the 2D image to achieve rigid registration for each bone of the multi-bone structure, so as to obtain a registration result for each bone. As shown in FIG. 10, a Bone 1 registration result, a Bone 2 registration result, ..., and a Bone N registration result may be obtained by respectively performing the rigid registration between each of the projection images DRR1, DRR2, ..., DRRN and the 2D image corresponding to the bone in the 2D images 1030. In some embodiments, a registration result for each bone refers to a target transformation matrix (also referred to as a registration matrix) corresponding to each bone, i.e., a transformation matrix from a 3D image coordinate system to an intraoperative surgical space coordinate system. By obtaining the registration matrix for each bone of the multi-bone structure as the registration result, the transformation of each bone can be reflected, which improves the accuracy and effectiveness of the registration.

In some embodiments, a preset threshold (e.g., the first preset threshold, the second preset threshold, etc.) of the similarity degree may be determined according to actual requirement(s). For instance, the preset threshold may be determined according to a registration part. For example, when registering the pelvis and the femur using the above registration process, and a gradient difference is designated as the similarity degree, the first preset threshold may be set to 0.5, the second preset threshold may be set to 0.88, the third preset threshold may be set to 0.2, and the fourth preset threshold may be set to 0.35. In some embodiments, since registration parts are different, and different similarity degree functions are used, the corresponding preset thresholds may also be different. For example, the first preset threshold may be within a range from 0.4 to 0.8, the second preset threshold may be within a range from 0.7 to 1.0, the third preset threshold may be within a range from 0.2 to 0.4, and the fourth preset threshold may be within a range from 0.3 to 0.5.

According to some embodiments of the present disclosure, the 3D image and the plurality of 2D images can be obtained. The first registered 3D image can be obtained by performing the spatial transformation on the 3D image based on the first spatial transformation parameter, and the first similarity degree between the first registered 3D image and the plurality of 2D images can be obtained. If the first similarity degree satisfies the first preset threshold, the single-bone 3D image of each bone can be obtained based on the first registered 3D image, and the single-bone 2D image of each bone can be obtained based on the plurality of 2D images. The second registered 3D image of each bone can be obtained by performing spatial transformation on the single-bone 3D image based on the second spatial transformation parameter, and the second similarity degree between the second registered 3D image and the corresponding single-bone 2D image can be obtained. If the second similarity degree satisfies the second preset threshold, the registration result can be obtained based on the second registered 3D image. The registration process using the overall rigid registration of the multi-bone structure combining with the rigid registration of single bone can improve the accuracy and the success rate of the registration algorithm. In addition, the above image registration process of the multi-bone structure needs no manual interaction, which can be automatically completed, thereby saving labor costs.

FIG. 11 is a structural schematic diagram illustrating a registration system according to some embodiments of the present disclosure.

In some embodiments, as shown in FIG. 11, the registration system may include a medical imaging device 1110, a processor 1120, and a navigation device 1130.

In some embodiments, the medical imaging device 1110 may be configured to capture at least one 2D image of a target object (e.g., a human body) during a surgery, and/or to capture a 3D image of the target object before the surgery, and send the at least one 2D image and/or the 3D image to the processor 1120. In some embodiments, the medical imaging device 1110 may include at least one of a 2D imaging device, a 3D imaging device, etc.

In some embodiments, the processor 1120 may be configured to obtain the 3D image of the target object captured before the surgery and the at least one 2D image captured during the surgery, and perform a 2D-3D registration on the 3D image and the at least one 2D image. For example, the processor 1120 may execute the registration process(es) described in FIGs. 3-10 of the present disclosure.

In some embodiments, the navigation device 1130 may be configured to navigate a surgical instrument to a target surgical region based on surgical guidance information. The target surgical region may correspond to a target part, such as a specific vertebra on a spine, etc. For example, the navigation device 1130 may transform surgical path information into the surgical guidance information based on a registration result (e.g., a target transformation matrix), so as to navigate the surgical instrument to the target surgical region. In some embodiments, the navigation device 1130 may include a navigation with doppler imaging (NDI) navigation system or other surgical navigation systems.

The possible beneficial effects of the embodiments of the present disclosure may include but not limited to the following: (1) improving existing 2D-3D registration techniques by automatically performing rough registration, automatically locating and extracting an anatomical structure region to be registered in 2D image(s), and only performing DRR projection on the anatomical structure region to be registered, no user interaction between preoperative and intraoperative images needs, which can reduce the interactive operations, and improve user experience and the clinical feasibility of the registration algorithm, thereby achieving a semiautomatic 2D-3D registration with minimal simple interaction and an automatic 2D-3D registration in certain registration scenarios; (2) by segmenting the 2D image(s) based on the 3D image, the disadvantage of low imaging definition of the 2D image(s) can be overcome using the advantage of the definition of the 3D image, and interference from a surgical device in the 2D image(s), etc., can be excluded, which can improve the accuracy of the segmentation, thereby ensuring the segmentation effect; (3) through the comprehensive use of the above techniques, fine mapping of target image(s) in the 2D image(s) to a surgical space coordinate system can be achieved, thereby ensuring the surgical effect. It should be noted that different embodiments may have different beneficial effects. In different embodiments, possible beneficial effects may be any one of the above effects, or any combination thereof, or any other beneficial effects that may be obtained.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended for those skilled in the art, though not expressly stated herein. These alterations, improvements, and modifications are intended to be suggested by this disclosure, and are within the scope of the exemplary embodiments of this disclosure.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this disclosure are not necessarily all referring to the same embodiment. Furthermore, the particular features, structures, or characteristics may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This method of disclosure, however, is not to be interpreted as reflecting an intention that the claimed subject matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate $\pm 20\%$ variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

By way of example, should there be any inconsistency or conflict between the description, definition, and/or the use of a term associated with the present document, the description, definition, and/or the use of the term in the present document shall prevail.

In closing, it is to be understood that the embodiments of the application disclosed herein are illustrative of the principles of the embodiments of the application. Other modifications that may be employed may be within the scope of the application. Thus, by way of example, but not of limitation, alternative configurations of the embodiments of the application may be utilized in accordance with the teachings herein. Accordingly, embodiments of the present application are not limited to that precisely as shown and described.

**Claims**

1. A method for registration, implemented by at least one processor (1120), the method comprising:

   obtaining a three-dimensional (3D) image of a target object captured before a surgery and at least one two-dimensional (2D) image captured during the surgery;
   obtaining a registered 3D image and a first transformation matrix between a 3D image coordinate system corresponding to the 3D image and a surgical space coordinate system corresponding to the surgery by performing, based on the at least one 2D image, posture transformation on the 3D image;
   determining a 3D target image corresponding to a target part in the registered 3D image;
   for each of the at least one 2D image, obtaining a fourth transformation matrix between a 2D image coordinate system corresponding to the 2D image and the surgical space coordinate system; and determining a 2D target image corresponding to the target part of the target object in each of the at least one 2D image based on the 3D target image, the first transformation matrix, and the fourth transformation matrix; and

obtaining a target transformation matrix by performing, based on the 3D target image and the 2D target image of each 2D image, posture transformation on the registered 3D image to optimize the first transformation matrix.

2. The method of claim 1, wherein the obtaining a registered 3D image and a first transformation matrix between a 3D image coordinate system corresponding to the 3D image and a surgical space coordinate system corresponding to the surgery by performing, based on the at least one 2D image, posture transformation on the 3D image includes:

obtaining a downsampled 3D image and at least one downsampled 2D image by downsampling, based on a preset multiplier, the 3D image and the at least one 2D image;
obtaining an adjusted downsampled 3D image by adjusting, based on a preset step size, a posture corresponding to the downsampled 3D image;
for each of the at least one 2D image, obtaining a first projection image corresponding to the 2D image by projecting, based on a capturing posture of the 2D image, the adjusted downsampled 3D image;
in response to determining that the at least one first projection image and the at least one downsampled 2D image satisfy a first preset condition, obtaining the registered 3D image by upsampling, based on the preset multiplier, the adjusted downsampled 3D image, wherein the first preset condition includes that a first similarity degree between the at least one first projection image and the at least one downsampled 2D image is larger than a similarity threshold; and
determining the first transformation matrix based on the posture transformation process from the 3D image to the registered 3D image.

3. The method of claim 2, wherein after for each of the at least one 2D image, obtaining the first projection image corresponding to the 2D image by projecting, based on the capturing posture of the 2D image, the adjusted downsampled 3D image, the method further comprises:

in response to determining that the at least one first projection image and the at least one downsampled 2D image do not satisfy the first preset condition, adjusting the posture corresponding to the adjusted downsampled 3D image based on the preset step size; and
repeating the operation of obtaining the first projection image corresponding to each of the at least one 2D image until the at least one first projection image and the at least one downsampled 2D image satisfy the first preset condition.

4. The method of claim 3, wherein in response to determining that the at least one first projection image and the at least one downsampled 2D image do not satisfy the first preset condition, the method further comprises:
adjusting the preset step size and/or the preset multiplier using a global optimizer.

5. The method of any one of claims 2-4, wherein the in response to determining that at least one first projection image and the at least one downsampled 2D image satisfy a first preset condition, obtaining the registered 3D image by upsampling, based on the preset multiplier, the adjusted downsampled 3D image includes:

in response to determining that the at least one first projection image and the at least one downsampled 2D image satisfy the first preset condition, and the preset multiplier does not satisfy a second preset condition, updating the 3D image to generate an updated 3D image by upsampling, based on the preset multiplier, the adjusted downsampled 3D image, wherein the second preset condition includes that the preset multiplier is equal to a preset value;
decreasing the preset multiplier; and
downsampling, based on the decreased preset multiplier, the updated 3D image and the at least one 2D image to repeat the operation of obtaining the first projection image corresponding to each of the at least one 2D image until the at least one first projection image and the at least one downsampled 2D image satisfy the first preset condition and the decreased preset multiplier satisfy the second preset condition.

6. The method of any one of claims 2-5, wherein the determining the first transformation matrix based on the posture transformation process from the 3D image to the registered 3D image includes:

obtaining a second transformation matrix between a 2D imaging device coordinate system and the surgical space coordinate system;
obtaining a third transformation matrix between the 3D image coordinate system and the 2D imaging device coordinate system based on the posture transformation process from the 3D image to the registered 3D image;

and
determining the first transformation matrix based on the second transformation matrix and the third transformation matrix.

7.  The method of claim 1, wherein for each of the at least one 2D image, the determining the 2D target image in the 2D image based on the 3D target image, the first transformation matrix, and the fourth transformation matrix includes:

    determining, based on the 3D target image, at least one 3D coordinate of at least one representative point of the target part in the 3D image coordinate system and a size parameter of the target part;
    determining at least one 2D coordinate of the at least one representative point in the 2D image coordinate system based on the at least one 3D coordinate, the first transformation matrix, and the fourth transformation matrix; and
    determining the 2D target image in the 2D image based on the at least one 2D coordinate and the size parameter.

8.  The method of claim 7, wherein the at least one representative point includes a center point of the target part in the 3D target image, and the size parameter is determined by:

    obtaining a second projection image corresponding to the 2D image by projecting, based on a capturing posture of the 2D image, the 3D target image;
    determining a center point of the target part in the second projection image by projecting the center point of the target part in the 3D target image;
    determining a distance collection as the size parameter, the distance collection including distances from the center point of the target part in the second projection image to the edges of the target part in the second projection image.

9.  The method of any one of claims 1 to 8, wherein the obtaining a fourth transformation matrix between a 2D image coordinate system corresponding to the 2D image and the surgical space coordinate system includes:

    obtaining a second transformation matrix between a 2D imaging device coordinate system and the surgical space coordinate system;
    obtaining a fifth transformation matrix between the 2D imaging device coordinate system and the 2D image coordinate system; and
    determining the fourth transformation matrix based on the second transformation matrix and the fifth transformation matrix.

10. The method of any one of claims 1-9, wherein the obtaining a target transformation matrix by performing, based on the 3D target image and the 2D target image of each 2D image, posture transformation on the registered 3D image to optimize the first transformation matrix includes:

    for each of the at least one 2D image, obtaining a second projection image corresponding to the 2D image by projecting, based on a capturing posture of the 2D image, the 3D target image;
    determining a second similarity degree based on the at least one second projection image and the at least one 2D target image; and
    obtaining the target transformation matrix by adjusting, based on the second similarity degree, a posture corresponding to the registered 3D image to optimize the first transformation matrix.

11. The method of claim 10, wherein the posture corresponding to the registered 3D image is adjusted iteratively until a third preset condition is satisfied, the third preset condition includes that a similarity difference between the second similarity degree and a last second similarity degree determined during the last adjustment of the posture corresponding to the registered 3D image is less than a threshold.

12. The method of claim 11, wherein after each time the posture corresponding to the registered 3D image is adjusted and an adjusted registered 3D image is generated, the method further comprises:

    determining whether the similarity difference is less than the threshold;
    in response to determining that the similarity difference is not less than the threshold, determining an adjustment step size based on the similarity difference, and
    adjusting the posture corresponding to the adjusted registered 3D image based on the adjustment step size.

13. A system (100) for registration comprising an image acquisition module (210), a posture transformation module (220), a target image determination module (230), and a target matrix determination module (240), wherein

the image acquisition module (210) is configured to obtain a three-dimensional (3D) image of a target object captured before a surgery and at least one two-dimensional (2D) image captured during the surgery;

the posture transformation module (220) is configured to obtain a registered 3D image and a first transformation matrix between a 3D image coordinate system corresponding to the 3D image and a surgical space coordinate system corresponding to the surgery by performing, based on the at least one 2D image, posture transformation on the 3D image;

the target image determination module (230) is configured to determine a 3D target image corresponding to a target part in the registered 3D image; for each of the at least one 2D image, obtain a fourth transformation matrix between a 2D image coordinate system corresponding to the 2D image and the surgical space coordinate system; and determine a 2D target image corresponding to the target part of the target object in each of the at least one 2D image based on the 3D target image, the first transformation matrix, and the fourth transformation matrix; and

the target matrix determination module (240) is configured to obtain a target transformation matrix by performing, based on the 3D target image and the 2D target image of each 2D image, posture transformation on the registered 3D image to optimize the first transformation matrix.

14. The system (100) of claim 13, wherein the posture transformation module (220) is further configured to:

obtain a downsampled 3D image and at least one downsampled 2D image by downsampling, based on a preset multiplier, the 3D image and the at least one 2D image;

obtain an adjusted downsampled 3D image by adjusting, based on a preset step size, a posture corresponding to the downsampled 3D image;

for each of the at least one 2D image, obtain a first projection image corresponding to the 2D image by projecting, based on a capturing posture of the 2D image, the adjusted downsampled 3D image;

in response to determining that the at least one first projection image and the at least one downsampled 2D image satisfy a first preset condition, obtain the registered 3D image by upsampling, based on the preset multiplier, the adjusted downsampled 3D image, wherein the first preset condition includes that a first similarity degree between the at least one first projection image and the at least one downsampled 2D image is larger than a similarity threshold; and

determine the first transformation matrix based on the posture transformation process from the 3D image to the registered 3D image.

15. A non-transitory computer-readable storage medium storing one or more computer instructions, wherein when a computer reads the one or more computer instructions from the storage medium, the computer executes the method according to claims 1-12.

**Patentansprüche**

1. Registrierungsverfahren, das durch mindestens einen Prozessor (1120) implementiert wird, wobei das Verfahren umfasst:

Erhalten eines dreidimensionalen (3D-) Bildes eines Zielobjektes, das vor einer Operation aufgenommen wird, und mindestens eines zweidimensionalen (2D-) Bildes, das während der Operation aufgenommen wird;

Erhalten eines registrierten 3D-Bildes und einer ersten Transformationsmatrix zwischen einem 3D-Bild-Koordinatensystem, das dem 3D-Bild entspricht, und einem Operationsraum-Koordinatensystem, das der Operation entspricht, durch Durchführen von Haltungstransformation in dem 3D-Bild auf Basis des mindestens einen 2D-Bildes;

Bestimmen eines 3D-Zielbildes, das einem Zielteil entspricht, in dem registrierten 3D-Bild;

Erhalten, für jedes des mindestens einen 2D-Bildes, einer vierten Transformationsmatrix zwischen einem 2D-Bild-Koordinatensystem, das dem 2D-Bild entspricht, und dem Operationsraum-Koordinatensystem; und Bestimmen eines 2D-Zielbildes, das dem Zielteil des Zielobjekts entspricht, in jedem des mindestens einen 2D-Bildes auf Basis des 3D-Zielbildes, der ersten Transformationsmatrix und der vierten Transformationsmatrix; und

Erhalten einer Zieltransformationsmatrix durch Durchführen von Haltungstransformation in dem registrierten 3D-Bild auf Basis des 3D-Zielbildes und des 2D-Zielbildes jedes 2D-Bildes, um die erste Transformationsmatrix zu optimieren.

2. Verfahren nach Anspruch 1, wobei das Erhalten eines registrierten 3D-Bildes und einer ersten Transformationsmatrix zwischen einem 3D-Bild-Koordinatensystem, das dem 3D-Bild entspricht, und einem Operationsraum-Koordinatensystem, das der Operation entspricht, durch Durchführen von Haltungstransformation in dem 3D-Bild auf Basis des mindestens einen 2D-Bildes beinhaltet:

Erhalten eines heruntergerechneten 3D-Bildes und mindestens eines heruntergerechneten 2D-Bildes durch Herunterrechnen des 3D-Bildes und des mindestens einen 2D-Bildes auf Basis eines voreingestellten Multiplikators;
Erhalten eines angepassten heruntergerechneten 3D-Bildes durch Anpassen einer Haltung, die dem heruntergerechneten 3D-Bild entspricht, auf Basis einer voreingestellten Schrittgröße;
Erhalten, für jedes des mindestens einen 2D-Bildes, eines ersten Projektionsbildes, das dem 2D-Bild entspricht, durch Projizieren des angepassten heruntergerechneten 3D-Bildes auf Basis einer Aufnahmehaltung des 2D-Bildes;
als Reaktion auf Bestimmen, dass das mindestens eine erste Projektionsbild und das mindestens eine heruntergerechnete 2D-Bild eine erste voreingestellte Bedingung erfüllen, Erhalten des registrierten 3D-Bildes durch Hochrechnen des angepassten heruntergerechneten 3D-Bildes auf Basis des voreingestellten Multiplikators, wobei die erste voreingestellte Bedingung beinhaltet, dass ein erster Ähnlichkeitsgrad zwischen dem mindestens einen ersten Projektionsbild und dem mindestens einen heruntergerechneten 2D-Bild größer ist als eine Ähnlichkeitsschwelle; und Bestimmen der ersten Transformationsmatrix auf Basis des Haltungstransformationsprozesses von dem 3D-Bild zu dem registrierten 3D-Bild.

3. Verfahren nach Anspruch 2, wobei das Verfahren nach dem Erhalten, für jedes des mindestens einen 2D-Bildes, des ersten Projektionsbildes, das dem 2D-Bild entspricht, durch Projizieren des angepassten heruntergerechneten 3D-Bildes auf Basis der Aufnahmehaltung des 2D-Bildes, weiter umfasst:

als Reaktion auf Bestimmen, dass das mindestens eine erste Projektionsbild und das mindestens eine heruntergerechnete 2D-Bild die erste voreingestellte Bedingung nicht erfüllen, Anpassen der Haltung, die dem angepassten heruntergerechneten 3D-Bild entspricht, auf Basis der voreingestellten Schrittgröße; und
Wiederholen des Vorgangs des Erhaltens des ersten Projektionsbildes, das jedem des mindestens einen 2D-Bildes entspricht, bis das mindestens eine erste Projektionsbild und das mindestens eine heruntergerechnete 2D-Bild die erste voreingestellte Bedingung erfüllen.

4. Verfahren nach Anspruch 3, wobei das Verfahren als Reaktion auf Bestimmen, dass das mindestens eine erste Projektionsbild und das mindestens eine heruntergerechnete 2D-Bild die erste voreingestellte Bedingung nicht erfüllen, weiter umfasst:
Anpassen der voreingestellten Schrittgröße und/oder des voreingestellten Multiplikators unter Verwendung eines globalen Optimierers.

5. Verfahren nach einem der Ansprüche 2-4, wobei Erhalten, als Reaktion auf Bestimmen, dass mindestens ein erstes Projektionsbild und das mindestens eine heruntergerechnete 2D-Bild eine erste voreingestellte Bedingung erfüllen, des registrierten 3D-Bildes durch Hochrechnen des angepassten heruntergerechneten 3D-Bildes auf Basis des voreingestellten Multiplikators beinhaltet:

als Reaktion auf Bestimmen, dass das mindestens eine erste Projektionsbild und das mindestens eine heruntergerechnete 2D-Bild die erste voreingestellte Bedingung erfüllen, und der voreingestellte Multiplikator eine zweite voreingestellte Bedingung nicht erfüllt, Aktualisieren des 3D-Bildes, um ein aktualisiertes 3D-Bild zu erzeugen, durch Hochrechnen des angepassten heruntergerechneten 3D-Bildes auf Basis des voreingestellten Multiplikators, wobei die zweite voreingestellte Bedingung beinhaltet, dass der voreingestellte Multiplikator gleich einem voreingestellten Wert ist;
Verringern des voreingestellten Multiplikators; und
Herunterrechnen des aktualisierten 3D-Bildes und des mindestens einen 2D-Bildes auf Basis des verringerten voreingestellten Multiplikators, um den Vorgang des Erhaltens des ersten Projektionsbildes, das jedem des mindestens einen 2D-Bildes entspricht, zu wiederholen, bis das mindestens eine erste Projektionsbild und das mindestens eine heruntergerechnete 2D-Bild die erste voreingestellte Bedingung erfüllen, und der verringerte voreingestellte Multiplikator die zweite voreingestellte Bedingung erfüllt.

6. Verfahren nach einem der Ansprüche 2-5, wobei Bestimmen der ersten Transformationsmatrix auf Basis des Haltungstransformationsprozesses von dem 3D-Bild zu dem registrierten 3D-Bild beinhaltet:

Erhalten einer zweiten Transformationsmatrix zwischen einem 2D-Bildgebungsvorrichtungs-Koordinatensystem und dem Operationsraum-Koordinatensystem;
Erhalten einer dritten Transformationsmatrix zwischen dem 3D-Bild-Koordinatensystem und dem 2D-Bildgebungsvorrichtungs-Koordinatensystem auf Basis des Haltungstransformationsprozesses von dem 3D-Bild zu dem registrierten 3D-Bild; und
Bestimmen der ersten Transformationsmatrix auf Basis der zweiten Transformationsmatrix und der dritten Transformationsmatrix.

7. Verfahren nach Anspruch 1, wobei Bestimmen, für jedes des mindestens einen 2D-Bildes, des 2D-Zielbildes in dem 2D-Bild auf Basis des 3D-Zielbildes, der ersten Transformationsmatrix und der vierten Transformationsmatrix beinhaltet:

Bestimmen mindestens einer 3D-Koordinate mindestens eines repräsentativen Punktes des Zielteils in dem 3D-Bild-Koordinatensystem und eines Größenparameters des Zielteils auf Basis des 3D-Zielbildes;
Bestimmen mindestens einer 2D-Koordinate des mindestens einen repräsentativen Punktes in dem 2D-Bild-Koordinatensystem auf Basis der mindestens einen 3D-Koordinate, der ersten Transformationsmatrix und der vierten Transformationsmatrix; und
Bestimmen des 2D-Zielbildes in dem 2D-Bild auf Basis der mindestens einen 2D-Koordinate und des Größenparameters.

8. Verfahren nach Anspruch 7, wobei der mindestens eine repräsentative Punkt einen Mittelpunkt des Zielteils in dem 3D-Zielbild beinhaltet, und der Größenparameter bestimmt wird durch:

Erhalten eines zweiten Projektionsbildes, das dem 2D-Bild entspricht, durch Projizieren des 3D-Zielbildes auf Basis einer Aufnahmehaltung des 2D-Bildes;
Bestimmen eines Mittelpunkts des Zielteils in dem zweiten Projektionsbild durch Projizieren des Mittelpunkts des Zielteils in dem 3D-Zielbild;
Bestimmen einer Abstandsmenge als den Größenparameter, wobei die Abstandsmenge Distanzen von dem Mittelpunkt des Zielteils in dem zweiten Projektionsbild zu den Rändern des Zielteils in dem zweiten Projektionsbild beinhaltet.

9. Verfahren nach einem der Ansprüche 1 bis 8, wobei das Erhalten einer vierten Transformationsmatrix zwischen einem 2D-Bild-Koordinatensystem, das dem 2D-Bild entspricht, und dem Operationsraum-Koordinatensystem beinhaltet:

Erhalten einer zweiten Transformationsmatrix zwischen einem 2D-Bildgebungsvorrichtungs-Koordinatensystem und dem Operationsraum-Koordinatensystem;
Erhalten einer fünften Transformationsmatrix zwischen dem 2D-Bildgebungsvorrichtungs-Koordinatensystem und dem 2D-Bild-Koordinatensystem; und
Bestimmen der vierten Transformationsmatrix auf Basis der zweiten Transformationsmatrix und der fünften Transformationsmatrix.

10. Verfahren nach einem der Ansprüche 1-9, wobei das Erhalten einer Zieltransformationsmatrix durch Durchführen von Haltungstransformation in dem registrierten 3D-Bild auf Basis des 3D-Zielbildes und des 2D-Zielbildes jedes 2D-Bildes, um die erste Transformationsmatrix zu optimieren, beinhaltet:

Erhalten, für jedes des mindestens einen 2D-Bildes, eines zweiten Projektionsbildes, das dem 2D-Bild entspricht, durch Projizieren des 3D-Zielbildes auf Basis einer Aufnahmehaltung des 2D-Bildes;
Bestimmen eines zweiten Ähnlichkeitsgrades auf Basis des mindestens einen zweiten Projektionsbildes und des mindestens einen 2D-Zielbildes; und
Erhalten der Zieltransformationsmatrix durch Anpassen einer Haltung, die dem registrierten 3D-Bild entspricht, auf Basis des zweiten Ähnlichkeitsgrades, um die erste Transformationsmatrix zu optimieren.

11. Verfahren nach Anspruch 10, wobei die Haltung, die dem registrierten 3D-Bild entspricht, iterativ angepasst wird, bis eine dritte voreingestellte Bedingung erfüllt ist, die dritte voreingestellte Bedingung beinhaltet, dass ein Ähnlichkeitsunterschied zwischen dem zweiten Ähnlichkeitsgrad und einem letzten zweiten Ähnlichkeitsgrad, der während der letzten Anpassung der Haltung, die dem registrierten 3D-Bild entspricht, bestimmt wurde, kleiner als eine Schwelle ist.

12. Verfahren nach Anspruch 11, wobei das Verfahren nach jedem Mal, bei dem die Haltung, die dem registrierten 3D-Bild entspricht, angepasst und ein angepasstes registriertes 3D-Bild erzeugt wird, weiter umfasst:

Bestimmen, ob der Ähnlichkeitsunterschied kleiner ist als die Schwelle;
als Reaktion auf Bestimmen, dass der Ähnlichkeitsunterschied nicht kleiner ist als die Schwelle, Bestimmen einer Anpassungsschrittgröße auf Basis des Ähnlichkeitsunterschiedes, und
Anpassen der Haltung, die dem angepassten registrierten 3D-Bild entspricht, auf Basis der Anpassungsschrittgröße.

13. Registrierungssystem (100), das ein Bildaufnahmemodul (210), ein Haltungstransformationsmodul (220), ein Zielbildbestimmungsmodul (230) und ein Zielmatrixbestimmungsmodul (240) umfasst, wobei

das Bildaufnahmemodul (210) so konfiguriert ist, dass es ein dreidimensionales (3D-) Bild eines Zielobjektes, das vor einer Operation aufgenommen wird, und mindestens ein zweidimensionales (2D-) Bild, das während der Operation aufgenommen wird, erhält;
das Haltungstransformationsmodul (220) so konfiguriert ist, dass es ein registriertes 3D-Bild und eine erste Transformationsmatrix zwischen einem 3D-Bild-Koordinatensystem, das dem 3D-Bild entspricht, und einem Operationsraum-Koordinatensystem, das der Operation entspricht, durch Durchführen von Haltungstransformation in dem 3D-Bild auf Basis des mindestens einen 2D-Bildes erhält;
das Zielbildbestimmungsmodul (230) so konfiguriert ist, dass es ein 3D-Zielbild, das einem Zielteil entspricht, in dem registrierten 3D-Bild bestimmt; für jedes des mindestens einen 2D-Bildes eine vierte Transformationsmatrix zwischen einem 2D-Bild-Koordinatensystem, das dem 2D-Bild entspricht, und dem Operationsraum-Koordinatensystem erhält; und ein 2D-Zielbild, das dem Zielteil des Zielobjekts entspricht, in jedem des mindestens einen 2D-Bildes auf Basis des 3D-Zielbildes, der ersten Transformationsmatrix und der vierten Transformationsmatrix bestimmt; und
das Zielmatrixbestimmungsmodul (240) so konfiguriert ist, dass es eine Zieltransformationsmatrix durch Durchführen von Haltungstransformation in dem registrierten 3D-Bild auf Basis des 3D-Zielbildes und des 2D-Zielbildes jedes 2D-Bildes erhält, um die erste Transformationsmatrix zu optimieren.

14. System (100) nach Anspruch 13, wobei das Haltungstransformationsmodul (220) weiter so konfiguriert ist, dass es:

ein heruntergerechnetes 3D-Bild und mindestens ein heruntergerechnetes 2D-Bild durch Herunterrechnen des 3D-Bildes und des mindestens einen 2D-Bildes auf Basis eines voreingestellten Multiplikators erhält;
ein angepasstes heruntergerechnetes 3D-Bild durch Anpassen einer Haltung, die dem heruntergerechneten 3D-Bild entspricht, auf Basis einer voreingestellten Schrittgröße erhält;
für jedes des mindestens einen 2D-Bildes ein erstes Projektionsbild, das dem 2D-Bild entspricht, durch Projizieren des angepassten heruntergerechneten 3D-Bildes auf Basis einer Aufnahmehaltung des 2D-Bildes erhält;
als Reaktion auf Bestimmen, dass das mindestens eine erste Projektionsbild und das mindestens eine heruntergerechnete 2D-Bild eine erste voreingestellte Bedingung erfüllen, das registrierte 3D-Bild durch Hochrechnen des angepassten heruntergerechneten 3D-Bildes auf Basis des voreingestellten Multiplikators erhält, wobei die erste voreingestellte Bedingung beinhaltet, dass ein erster Ähnlichkeitsgrad zwischen dem mindestens einen ersten Projektionsbild und dem mindestens einen heruntergerechneten 2D-Bild größer ist als eine Ähnlichkeitsschwelle; und
die erste Transformationsmatrix auf Basis des Haltungstransformationsprozesses von dem 3D-Bild zu dem registrierten 3D-Bild bestimmt.

15. Nichtflüchtiges computerlesbares Speichermedium, das eine oder mehrere Computeranweisungen speichert, wobei, wenn ein Computer die eine oder die mehreren Computeranweisungen von dem Speichermedium liest, der Computer das Verfahren nach den Ansprüchen 1-12 ausführt.

**Revendications**

1. Procédé d'enregistrement, mis en œuvre par au moins un processeur (1120), le procédé comprenant :

   l'obtention d'une image tridimensionnelle (3D) d'un objet cible capturée avant une intervention chirurgicale et au moins une image bidimensionnelle (2D) capturée pendant l'intervention chirurgicale ;
   l'obtention d'une image 3D enregistrée et d'une première matrice de transformation entre un système de coordonnées d'image 3D correspondant à l'image 3D et un système de coordonnées d'espace chirurgical correspondant à l'intervention chirurgicale en effectuant, sur la base de l'au moins une image 2D, une transformation de posture sur l'image 3D ;
   la détermination d'une image cible 3D correspondant à une partie cible dans l'image 3D enregistrée ;
   pour chacune de l'au moins une image 2D, l'obtention d'une quatrième matrice de transformation entre un système de coordonnées d'image 2D correspondant à l'image 2D et le système de coordonnées d'espace chirurgical ; et la détermination d'une image cible 2D correspondant à la partie cible de l'objet cible dans chacune de l'au moins une image 2D sur la base de l'image cible 3D, de la première matrice de transformation et de la quatrième matrice de transformation ; et
   l'obtention d'une matrice de transformation cible en effectuant, sur la base de l'image cible 3D et de l'image cible 2D de chaque image 2D, une transformation de posture sur l'image 3D enregistrée pour optimiser la première matrice de transformation.

2. Procédé selon la revendication 1, dans lequel l'obtention d'une image 3D enregistrée et d'une première matrice de transformation entre un système de coordonnées d'image 3D correspondant à l'image 3D et un système de coordonnées d'espace chirurgical correspondant à l'intervention chirurgicale en effectuant, sur la base de l'au moins une image 2D, une transformation de posture sur l'image 3D inclut :

   l'obtention d'une image 3D sous-échantillonnée et d'au moins une image 2D sous-échantillonnée par sous-échantillonnage, sur la base d'un multiplicateur prédéfini, de l'image 3D et de l'au moins une image 2D ;
   l'obtention d'une image 3D sous-échantillonnée ajustée en ajustant, sur la base d'une taille de pas prédéfinie, une posture correspondant à l'image 3D sous-échantillonnée ;
   pour chacune de l'au moins une image 2D, l'obtention d'une première image de projection correspondant à l'image 2D en projetant, sur la base d'une posture de capture de l'image 2D, l'image 3D sous-échantillonnée ajustée ;
   en réponse à la détermination que l'au moins une première image de projection et l'au moins une image 2D sous-échantillonnée satisfont à une première condition prédéfinie, l'obtention de l'image 3D enregistrée par suréchantillonnage, sur la base du multiplicateur prédéfini, de l'image 3D sous-échantillonnée ajustée, dans lequel la première condition prédéfinie inclut qu'un premier degré de similarité entre l'au moins une première image de projection et l'au moins une image 2D sous-échantillonnée est supérieur à un seuil de similarité ; et la détermination de la première matrice de transformation sur la base du processus de transformation de posture de l'image 3D à l'image 3D enregistrée.

3. Procédé selon la revendication 2, dans lequel, après avoir obtenu, pour chacune de l'au moins une image 2D, la première image de projection correspondant à l'image 2D en projetant, sur la base de la posture de capture de l'image 2D, l'image 3D sous-échantillonnée ajustée, le procédé comprend en outre :

   en réponse à la constatation que l'au moins une première image de projection et l'au moins une image 2D sous-échantillonnée ne satisfont pas à la première condition prédéfinie, l'ajustement de la posture correspondant à l'image 3D sous-échantillonnée ajustée en fonction de la taille de pas prédéfinie ; et
   la répétition de l'opération d'obtention de la première image de projection correspondant à chacune de l'au moins une image 2D jusqu'à ce que l'au moins une première image de projection et l'au moins une image 2D sous-échantillonnée satisfassent à la première condition prédéfinie.

4. Procédé selon la revendication 3, dans lequel, en réponse à la détermination que l'au moins une première image de projection et l'au moins une image 2D sous-échantillonnée ne satisfont pas à la première condition prédéfinie, le procédé comprend en outre :
   l'ajustement de la taille de pas prédéfinie et/ou du multiplicateur prédéfini utilisant un optimiseur global.

5. Procédé selon l'une quelconque des revendications 2-4, dans lequel, en réponse à la détermination qu'au moins une première image de projection et l'au moins une image 2D sous-échantillonnée satisfont à une première condition prédéfinie, l'obtention de l'image 3D enregistrée par suréchantillonnage, sur la base du multiplicateur prédéfini, de l'image 3D sous-échantillonnée ajustée inclut :

en réponse à la détermination que l'au moins une première image de projection et l'au moins une image 2D sous-échantillonnée satisfont à la première condition prédéfinie, et que le multiplicateur prédéfini ne satisfait pas à une deuxième condition prédéfinie, la mise à jour de l'image 3D pour générer une image 3D mise à jour par suréchantillonnage, sur la base du multiplicateur prédéfini, de l'image 3D sous-échantillonnée ajustée, dans lequel la deuxième condition prédéfinie inclut que le multiplicateur prédéfini est égal à une valeur prédéfinie ; la diminution du multiplicateur prédéfini ; et

le sous-échantillonnage, sur la base du multiplicateur prédéfini diminué, de l'image 3D mise à jour et de l'au moins une image 2D pour répéter l'opération d'obtention de la première image de projection correspondant à chacune de l'au moins une image 2D jusqu'à ce qu'au moins une première image de projection et l'au moins une image 2D sous-échantillonnée satisfassent à la première condition prédéfinie et que le multiplicateur prédéfini diminué satisfasse à la deuxième condition prédéfinie.

6. Procédé selon l'une quelconque des revendications 2-5, dans lequel la détermination de la première matrice de transformation sur la base du processus de transformation de posture de l'image 3D à l'image 3D enregistrée inclut :

l'obtention d'une seconde matrice de transformation entre un système de coordonnées de dispositif d'imagerie 2D et le système de coordonnées de l'espace chirurgical ;

l'obtention d'une troisième matrice de transformation entre le système de coordonnées d'image 3D et le système de coordonnées de dispositif d'imagerie 2D, sur la base du processus de transformation de posture de l'image 3D à l'image 3D enregistrée ; et

la détermination de la première matrice de transformation sur la base de la deuxième matrice de transformation et de la troisième matrice de transformation.

7. Procédé selon la revendication 1, dans lequel pour chacune de l'au moins une image 2D, la détermination de l'image cible 2D dans l'image 2D sur la base de l'image cible 3D, la première matrice de transformation et la quatrième matrice de transformation inclut :

la détermination, à partir de l'image cible 3D, d'au moins une coordonnée 3D d'au moins un point représentatif de la partie cible dans le système de coordonnées d'image 3D et un paramètre de taille de la partie cible ;

la détermination d'au moins une coordonnée 2D de l'au moins un point représentatif dans le système de coordonnées d'image 2D sur la base de l'au moins une coordonnée 3D, de la première matrice de transformation et de la quatrième matrice de transformation ; et

la détermination de l'image cible 2D dans l'image 2D sur la base de l'au moins une coordonnée 2D et du paramètre de taille.

8. Procédé selon la revendication 7, dans lequel l'au moins un point représentatif inclut un point central de la partie cible dans l'image cible 3D et le paramètre de taille est déterminé par :

l'obtention d'une seconde image de projection correspondant à l'image 2D en projetant, sur la base d'une posture de capture de l'image 2D, l'image cible 3D ;

la détermination d'un point central de la pièce cible dans la deuxième image de projection en projetant le point central de la pièce cible dans l'image cible 3D ;

la détermination d'une collection de distances comme paramètre de taille, la collection de distances incluant des distances entre le point central de la partie cible dans la deuxième image de projection et les bords de la partie cible dans la deuxième image de projection.

9. Procédé selon l'une quelconque des revendications 1 à 8, dans lequel l'obtention d'une quatrième matrice de transformation entre un système de coordonnées d'image 2D correspondant à l'image 2D et le système de coordonnées d'espace chirurgical inclut :

l'obtention d'une seconde matrice de transformation entre le système de coordonnées de dispositif d'imagerie 2D et le système de coordonnées d'espace chirurgical ;

l'obtention d'une cinquième matrice de transformation entre le système de coordonnées de dispositif d'imagerie 2D et le système de coordonnées de l'image 2D ; et

la détermination de la quatrième matrice de transformation sur la base de la deuxième matrice de transformation et de la cinquième matrice de transformation.

10. Procédé selon l'une quelconque des revendications 1-9, dans lequel l'obtention d'une matrice de transformation cible en effectuant, sur la base de l'image cible 3D et de l'image cible 2D de chaque image 2D, une transformation de posture sur l'image 3D enregistrée pour optimiser la première matrice de transformation inclut :

pour chacune de l'au moins une image 2D, l'obtention d'une seconde image de projection correspondant à l'image 2D en projetant, sur la base d'une posture de capture de l'image 2D, l'image cible 3D ;

la détermination d'un second degré de similarité sur la base de l'au moins une seconde image de projection et de l'au moins une image cible 2D ; et

l'obtention de la matrice de transformation cible en ajustant, sur la base du deuxième degré de similarité, une posture correspondant à l'image 3D enregistrée pour optimiser la première matrice de transformation.

11. Procédé selon la revendication 10, dans lequel la posture correspondant à l'image 3D enregistrée est ajustée de manière itérative jusqu'à ce qu'une troisième condition prédéfinie soit satisfaite, la troisième condition prédéfinie inclut qu'une différence de similarité entre le deuxième degré de similarité et un dernier deuxième degré de similarité déterminé pendant le dernier ajustement de la posture correspondant à l'image 3D enregistrée est inférieure à un seuil.

12. Procédé selon la revendication 11, dans lequel, après chaque ajustement de la posture correspondant à l'image 3D enregistrée et génération d'une image 3D enregistrée ajustée, le procédé comprend en outre :

la détermination si la différence de similarité est inférieure au seuil ;

en réponse à la détermination que la différence de similarité n'est pas inférieure au seuil, la détermination d'une taille de pas d'ajustement sur la base de la différence de similarité, et

l'ajustement de la posture correspondant à l'image 3D enregistrée ajustée sur la base de la taille de pas d'ajustement.

13. Système (100) d'enregistrement comprenant un module d'acquisition d'images (210), un module de transformation de posture (220), un module de détermination d'image cible (230) et un module de détermination de matrice cible (240), dans lequel

le module d'acquisition d'images (210) est configuré pour obtenir une image tridimensionnelle (3D) d'un objet cible capturé avant une intervention chirurgicale et au moins une image bidimensionnelle (2D) capturée pendant l'intervention chirurgicale ;

le module de transformation de posture (220) est configuré pour obtenir une image 3D enregistrée et une première matrice de transformation entre un système de coordonnées d'image 3D correspondant à l'image 3D et un système de coordonnées d'espace chirurgical correspondant à l'intervention chirurgicale en effectuant, sur la base de l'au moins une image 2D, une transformation de posture sur l'image 3D ;

le module de détermination de l'image cible (230) est configuré pour déterminer une image cible 3D correspondant à une partie cible dans l'image 3D enregistrée ; pour chacune de l'au moins une image 2D, obtenir une quatrième matrice de transformation entre un système de coordonnées d'image 2D correspondant à l'image 2D et le système de coordonnées d'espace chirurgical ; et déterminer une image cible 2D correspondant à la partie cible de l'objet cible dans chacune de l'au moins une image 2D sur la base de l'image cible 3D, de la première matrice de transformation et de la quatrième matrice de transformation ; et

le module de détermination de matrice cible (240) est configuré pour obtenir une matrice de transformation cible en effectuant, sur la base de l'image cible 3D et de l'image cible 2D de chaque image 2D, une transformation de posture sur l'image 3D enregistrée pour optimiser la première matrice de transformation.

14. Système (100) selon la revendication 13, dans lequel le module de transformation de posture (220) est en outre configuré pour :

obtenir une image 3D sous-échantillonnée et au moins une image 2D sous-échantillonnée en sous-échantillonnant, sur la base d'un multiplicateur prédéfini, l'image 3D et au moins une image 2D ;

obtenir une image 3D sous-échantillonnée ajustée en ajustant, sur la base d'une taille de pas prédéfinie, une posture correspondant à l'image 3D sous-échantillonnée ;

pour chacune de l'au moins une image 2D, obtenir une première image de projection correspondant à l'image 2D en projetant, sur la base d'une posture de capture de l'image 2D, l'image 3D sous-échantillonnée ajustée ;

en réponse à la détermination que l'au moins une première image de projection et l'au moins une image 2D sous-échantillonnée satisfont à une première condition prédéfinie, obtenir l'image 3D enregistrée par suréchantillonnage, sur la base du multiplicateur prédéfini, de l'image 3D sous-échantillonnée ajustée, dans lequel la première condition prédéfinie inclut qu'un premier degré de similarité entre l'au moins une première image de projection et l'au moins une image 2D sous-échantillonnée est supérieur à un seuil de similarité ; et
déterminer la première matrice de transformation sur la base du processus de transformation de posture de l'image 3D à l'image 3D enregistrée.

15. Support de stockage non transitoire lisible par ordinateur stockant une ou plusieurs instructions informatiques, dans lequel lorsqu'un ordinateur lit les une ou plusieurs instructions informatiques à partir du support de stockage, l'ordinateur exécute le procédé selon les revendications 1-12.

100

FIG. 1

Image Acquisition Module
210

Posture Transformation Module
220

Target Image Determination
Module 230

Target Matrix Determination
Module 240

FIG. 2

**300**

Obtaining a three-dimensional (3D) image of a target object captured before a surgery and at least one two-dimensional (2D) image captured during the surgery — 310

Obtaining a registered 3D image and a first transformation matrix between a 3D image coordinate system corresponding to the 3D image and a surgical space coordinate system corresponding to the surgery by performing posture transformation on the 3D image based on the at least one 2D image — 320

Determining a 2D target image corresponding to a target part of the target object in each of the at least one 2D image and determining a 3D target image corresponding to the target part in the registered 3D image — 330

Obtaining a target transformation matrix by performing, based on the 3D target image and the 2D target image of each 2D image, posture transformation on the registered 3D image to optimize the first transformation matrix — 340

**FIG. 3**

**400**

410

Obtaining a downsampled 3D image and at least one downsampled 2D image by downsampling a 3D image and at least one 2D image based on a preset multiplier

420

Obtaining an adjusted downsampled 3D image by adjusting, based on a preset step size, a posture corresponding to the downsampled 3D image

430

For each of the at least one 2D image, obtaining a first projection image corresponding to the 2D image by projecting the adjusted downsampled 3D image based on a capturing posture of the 2D image

440

Determining a first similarity degree based on the at least one first projection image and the at least one downsampled 2D image

450

In response to determining that the first similarity degree is larger than a similarity threshold, determining that the at least one first projection image and the at least one downsampled 2D image satisfy a first preset condition

460

In response to determining that the at least one first projection image and the at least one downsampled 2D image satisfy the first preset condition, obtaining a registered 3D image by upsampling the adjusted downsampled 3D image based on a preset multiplier

470

Determining a first transformation matrix based on the posture transformation process from the 3D image to the registered 3D image

**FIG. 4**

**500**

510
Determining a 3D target image corresponding to a target part in a registered 3D image

520
Obtaining a fourth transformation matrix between a 2D image coordinate system corresponding to a 2D image and a surgical space coordinate system

530
Determining, based on the 3D target image, at least one 3D coordinate of at least one representative point of the target part in a 3D image coordinate system and a size parameter of the target part

540
Determining at least one 2D coordinate of the at least one representative point in the 2D image coordinate system based on the at least one 3D coordinate, a first transformation matrix, and a fourth transformation matrix

550
Determining a 2D target image in the 2D image based on the at least one 2D coordinate and the size parameter

FIG. 5

**600**

610
For each of at least one 2D image, obtaining a second projection image corresponding to the 2D image by projecting, based on a capturing posture of the 2D image, a 3D target image

620
Determining a second similarity degree based on the at least one second projection image and at least one 2D target image

630
Obtaining a target transformation matrix by adjusting, based on the second similarity degree, a posture corresponding to a registered 3D image to optimize a first transformation matrix

FIG. 6

FIG. 7A

FIG. 7B

FIG. 8A

FIG. 8B

901 Obtaining a 3D image Y0 of the spine captured before a surgery

903 Obtaining a 2D image Y1 captured at a first capturing posture and a 2D image Y2 captured at a second capturing posture during the surgery

902 Obtaining a segmentation mask for each vertebra and a label for the segmentation mask by preprocessing Y0

904 Determining a fourth transformation matrix T1a corresponding to Y1 and a fourth transformation matrix T1b corresponding to Y2

905 Obtaining a first transformation matrix T2 between a 3D image coordinate system and a surgical space coordinate system, and a registered 3D image Y4 by performing a rough registration based Y0, Y1, and Y2

906 Designating a label of a target part to determine a segmentation mask for the target part and determining, based on the segmentation mask for the part, a 3D target image y0 in Y4 and a first center point s1 of the target part

907 Obtaining a second projection image y01 and a second projection image y02 by projecting y0 at a first projection posture and a second projection posture, respectively

908 Determining a minimal enclosing rectangle rec1 of the target part in y01 and a minimal enclosing rectangle rec2 of the target part in y02

909 Transforming s1 into the surgical space coordinate system to s2 based on T2, transforming s2 into a 2D image coordinate system corresponding to Y1 based on T1a to obtain s3, transforming s2 into a 2D image coordinate system corresponding to Y2 based on T1b to obtain s4

910 Determining a 2D target image y1 corresponding to the target part in Y1 based on s3 and rec1, and a 2D target image y2 corresponding to the target part in Y2 based on s4 and rec2

911 Determining a second similarity degree based on y1, y2, y01, and y02

912 The second similarity degree satisfies a third preset condition?

No

914 i = i + 1, adjusting a possure of Y4 based on the second similarity degree

913 Obtaining a current iteration count i and determining whether i satisfies a fourth preset condition

Yes

No

Yes

915 Obtaining a target transformation matrix

FIG. 9

```
┌─────────────────────┐
│ 3D image of a multi-bone │
│  structure 1010     │
└─────────────────────┘
           │
           ▼
┌─────────────────────┐
│     DRR image       │
│       1020          │
└─────────────────────┘
```

```
┌──────────────────────┐
│  Overall rigid        │
│  registration of the  │
│  multi-bone           │
│  structure 1040       │
└──────────────────────┘
           │
           ▼
┌──────────────────────────┐
│ Automatic bone segmentation │
│          1050             │
└──────────────────────────┘
```

```
┌──────────────┐
│  2D image     │
│    1030       │
└──────────────┘
```

```
┌──────────┐  ┌──────────┐       ┌──────────┐
│  Bone 1  │  │  Bone 2  │  ···  │  Bone N  │
└──────────┘  └──────────┘       └──────────┘
     │             │                  │
     ▼             ▼                  ▼
┌──────────┐  ┌──────────┐       ┌──────────┐
│  DRR 1   │  │  DRR 2   │  ···  │  DRR N   │
└──────────┘  └──────────┘       └──────────┘
```

```
┌──────────────┐  ┌──────────────┐       ┌──────────────┐
│   Bone 1     │  │   Bone 2     │  ···  │   Bone N     │
│   Rigid      │  │   Rigid      │       │   Rigid      │
│ registration │  │ registration │       │ registration │
└──────────────┘  └──────────────┘       └──────────────┘
       │                 │                       │
       ▼                 ▼                       ▼
┌──────────────┐  ┌──────────────┐       ┌──────────────┐
│   Bone 1     │  │   Bone 2     │  ···  │   Bone N     │
│ Rigid result │  │ Rigid result │       │ Rigid result │
└──────────────┘  └──────────────┘       └──────────────┘
```

**FIG. 10**

```
        1110                  1120                  1130
         ∿                     ∿                     ∿
┌──────────────────────┐  ┌──────────┐  ┌──────────────────────┐
│ Medical imaging device │──│ processor │──│  Navigation device   │
└──────────────────────┘  └──────────┘  └──────────────────────┘
```

**FIG. 11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210147197

- CN 202110875674

**Non-patent literature cited in the description**

- **KETCHA M. D. et al.** *Multi-stage 3D-2D registration for correction of anatomical deformation in image-guided spine surgery*